# EUROPEAN PATENT APPLICATION

(11) **EP 3 915 567 A1**
(43) Date of publication of application: **01.12.2021**
(21) Application number: 19912022.1
(22) Date of filing: 25.01.2019
(51) Int. Cl.: A61K 31/7105, A61P 1/16, A61P 11/00, A61P 13/12

(54) **THERAPEUTIC AGENT FOR FIBROSIS**

(71) Applicant: Kyorin Pharmaceutical Co., Ltd., Tokyo 101-8311 (JP)
(72) Inventor: NAKAO, Kazuhisa, Shimotsuga-gun, Tochigi 329-0114 (JP); ISHIYAMA, Junichi, Tokyo 101-8311 (JP); ICHIKAWA, Wataru, Shimotsuga-gun, Tochigi 329-0114 (JP); MASUI ,Atsushi, Shimotsuga-gun, Tochigi 329-0114 (JP); AKASAKA, Yunike, Shimotsuga-gun, Tochigi 329-0114 (JP); HONDA, Aya, Shimotsuga-gun, Tochigi 329-0114 (JP)
(74) Representative: Handley, Matthew Edward
(86) International application number: PCT/JP2019/002560
(87) International publication number: WO 2020/152869

(57) **Abstract**

Disclosed is an antisense nucleic acid that suppresses NEK6 gene expression.

SEQUENCE LISTING

<110>  Kyorin pharmaceutical co.,ltd

<120>  THERAPEUTIC AGENT FOR FIBROSIS

<130>  FP18-1358-00

<160>  17

<170>  PatentIn version 3.5

<210>  1
<211>  16
<212>  DNA
<213>  Artificial Sequence

<220>
<223>  KB-XAX

<400>  1
gcaatcctga aggtag                                                    16

<210>  2
<211>  16
<212>  DNA
<213>  Artificial Sequence

<220>
<223>  KB-XBX

<400>  2
gacagctcag acaatt                                                    16

<210>  3
<211>  16
<212>  DNA
<213>  Artificial Sequence

<220>
<223>  KB-XCX

<400>  3
tcaaggaggt gacgaa                                                    16

<210>  4
<211>  2645
<212>  DNA
<213>  Homo sapiens

<400>  4
gggcgggcgc gcgggcgcgc gggcccgcgc aggcggtggc ggcggcggcg gaaccgagct      60

gacgggcgtg cggccgctgc gccgcaaact cgtgtgggac gcaccgctcc agccgcccgc     120

gggccagcgc accggtcccc cagcggcagc cgagcccgcc cgcgcgccgt tcgtgccctc     180

gtgaggctgg catgcaggat ggcaggacag cccggccaca tgccccatgg agggagttcc     240

aacaacctct gccacaccct ggggcctgtg catcctcctg acccacagag gcatcccaac     300

acgctgtctt ttcgctgctc gctggcggac ttccagatcg aaaagaagat aggccgagga 360
cagttcagcg aggtgtacaa ggccacctgc ctgctggaca ggaagacagt ggctctgaag 420
aaggtgcaga tctttgagat gatggacgcc aaggcgaggc aggactgtgt caaggagatc 480
ggcctcttga agcaactgaa ccacccaaat atcatcaagt atttggactc gtttatcgaa 540
gacaacgagc tgaacattgt gctggagttg gctgacgcag gggacctctc gcagatgatc 600
aagtacttta agaagcagaa gcggctcatc ccggagagga cagtatggaa gtactttgtg 660
cagctgtgca gcgccgtgga gcacatgcat tcacgccggg tgatgcaccg agacatcaag 720
cctgccaacg tgttcatcac agccacgggc gtcgtgaagc tcggtgacct tggtctgggc 780
cgcttcttca gctctgagac caccgcagcc cactccctag tggggacgcc ctactacatg 840
tcaccggaga ggatccatga gaacggctac aacttcaagt ccgacatctg gtccctgggc 900
tgtctgctgt acgagatggc agccctccag agccccttct atggagataa gatgaatctc 960
ttctccctgt gccagaagat cgagcagtgt gactaccccc cactccccgg ggagcactac 1020
tccgagaagt tacgagaact ggtcagcatg tgcatctgcc ctgaccccca ccagagacct 1080
gacatcggat acgtgcacca ggtggccaag cagatgcaca tctggatgtc cagcacctga 1140
gcgtggatgc accgtgcctt atcaaagcca gcaccacttt gccttacttg agtcgtcttc 1200
tcttcgagtg gccacctggt agcctagaac agctaagacc acagggttca gcaggttccc 1260
caaaaggctg cccagcctta cagcagatgc tgaaggcaga gcagctgagg gaggggcgct 1320
ggccacatgt cactgatggt cagattccaa agtcctttct ttatactgtt gtggacaatc 1380
tcagctgggt caataagggc aggtggttca gcgagccacg gcagccccct gtatctggat 1440
tgtaatgtga atctttaggg taattcctcc agtgacctgt caaggcttat gctaacagga 1500
gacttgcagg agaccgtgtg atttgtgtag tgagcctttg aaaatggtta gtaccgggtt 1560
cagtttagtt cttagtatct tttcaatcaa gctgtgtgct taatttactc tgttgtaaag 1620
ggataaagtg gaaatcattt ttttccgtgg agtggtgatt ctgctaacat ttttatctac 1680
gttttataac ttggtgagtg acgatgagag ccctgcacct ggccagagtg tcacaggcaa 1740
aaggcatcgg gaagcaggag catcttcttg gcagccaggc tgggccatct tctcctggac 1800
acctgctgtg taccaggaac ttcgtcacct ccttgaatgc tggcggttca tttcatgatc 1860
agtgttaagc attttcctcc atgggaagga agcatgggat atagaaaagc gaagggctgt 1920
cctttacaaa ttctggttct gcaacttcct agcgtgactt tgggcttggg caagtttctt 1980
agccgttctg agccttcatt tcctcatctg tacaatgaga ttaatagtac ctatcatcta 2040
ccttcaggat tgctgacaga cagaatttga aataaaatat gcaagttagc taatacaaaa 2100
agtagatgat ccaaaaatgg tagccactca cccttcacaa actgaagtcc atggaccacg 2160

gaagtcgaga attaatgtac acctgtatca tgtgtaggaa accagaaatg tgttccttat 2220

ttcttgttcc caaacaggat taactgtgaa gactaattta taaatgtgaa cctaagaaaa 2280

ctccacctct gaaggaaatc atttgaattt tgtttttgta cgtaaagtta accttccaat 2340

tgtctgagct gtcgtcactg acttcatgac agtctggccc tccagacaag agcagcgctg 2400

gcatcgggca ggtgattcct gacacctgct gcctgcaggc attcactgac caggcctttc 2460

ctggaggaaa cacccagggc cgggcggctg ctgtttccac acgtggactc ggatctgctg 2520

tgacaccgtc agcccgacag tctctccata tgcagccttt cctctgtact tttctccatg 2580

gttgaaataa aacagggtga ctgggagtta cttagaattc atgaagattt taaaaaaaaa 2640

aaaaa 2645

<210> 5
<211> 25
<212> DNA
<213> Artificial Sequence

<220>
<223> 18s MGB Probe

<400> 5
attggagggc aagtctggtg ccagc 25

<210> 6
<211> 21
<212> DNA
<213> Artificial Sequence

<220>
<223> Primer 1

<400> 6
cggctaccac atccaaggaa g 21

<210> 7
<211> 18
<212> DNA
<213> Artificial Sequence

<220>
<223> Primer 2

<400> 7
gctggaatta ccgcggct 18

<210> 8
<211> 48
<212> DNA
<213> Artificial Sequence

<220>
<223> pTL-SBE-luc

<400>  8
agtatgtcta gactgaagta tgtctagact gaagtatgtc tagactga                    48

<210> 9
<211> 19
<212> RNA
<213> Artificial

<220>
<223> Synthetic RNA

<400> 9
cuguccucgg ccuaucuuc                                                    19

<210> 10
<211> 19
<212> RNA
<213> Artificial

<220>
<223> Synthetic RNA

<400> 10
uauuugggug guucaguug                                                    19

<210> 11
<211> 19
<212> RNA
<213> Artificial

<220>
<223> Synthetic RNA

<400> 11
caacuccagc acaauguuc                                                    19

<210> 12
<211> 19
<212> RNA
<213> Artificial

<220>
<223> Synthetic RNA

<400> 12
uacuugauca ucugcgaga                                                    19

<210> 13
<211> 48
<212> RNA
<213> Artificial Sequence

<220>
<223> KB-001

<400> 13
gagggaguuc caacaaccuc uccggagagg uuguuggaac ucccucca                    48

<210> 14
<211> 48
<212> RNA

<213> Artificial Sequence

<220>
<223> KB-002

<400> 14
cgaggcagga cugugucaag gccggccuug acacaguccu gccucgcc 48

<210> 15
<211> 48
<212> RNA
<213> Artificial Sequence

<220>
<223> KB-003

<400> 15
cguggagcac augcauucac gccggcguga augcaugugc uccacggc 48

<210> 16
<211> 48
<212> RNA
<213> Artificial Sequence

<220>
<223> KB-004

<400> 16
gauaagauga aucucuucuc cccggggaga agagauucau cuuaucuc 48

<210> 17
<211> 48
<212> RNA
<213> Artificial Sequence

<220>
<223> KB-005

<400> 17
cagagaccug acaucggaua cccggguauc cgaugucagg ucucuggu 48

## Description

### Technical Field

The present invention relates to an antisense nucleic acid that suppresses NEK6 gene expression and a medicament comprising the nucleic acid as an active ingredient.

### Background Art

Fibrosis is a disease in which organ function is impaired with excessive accumulation of collagen to lead to irreversible progression, and for example, skin, lung, liver, pancreas, kidney, bone marrow, and the like has been known as the onset organs. Idiopathic pulmonary fibrosis (IPF), which is a type of fibrosis in the lung, is designated as an intractable disease in Japan because, although the disease prevalence is not high as represented by about twenty patients per one hundred thousand population, the post-treatment prognosis is undesirable as represented by the average survival period of 2.5 to 5 years after confirmation of diagnosis.

As therapeutic drugs for IPF, pirfenidon and nintedanib were approved by the Japanese Ministry of Health, Labour and Welfare and have been launched to date. Although both drugs show suppression of decline in vital capacity and prolongation action on progression-free survival and slow down progression of pathological condition, these cannot be deemed to be sufficiently satisfactory as therapeutic efficacies, and development of a therapeutic drug based on a new mechanism has been demanded.

Meanwhile, "NEK6 protein", which is a target of the present invention, is known as one of NIMA-related serine/threonine kinase family involved in control of cell division, but has not drawn attention as a research subject for drug development to date. Although it was reported to have potential as a drug development target for cancer in 2002, there is no specific report that NEK6 protein interacts with SMAD2/3 protein and promotes tissue fibrogenesis.

### Citation List

### Patent Literature

Patent Literature 1: U.S. Patent Application Publication No. 2004/0097441

### Summary of Invention

### Technical Problem

The present invention has an object to provide a novel phosphorylation inhibitor of SMAD2/3 protein and a therapeutic agent for fibrosis.

### Solution to Problem

The inventors focused attention on finding of enhancement of mRNA level of NEK6 (NIMA-related serine/threonine kinase 6) from analysis with a bleomycin-induced pulmonary fibrosis model, then proceeded with their research, and as a result, found that NEK6 controls SMAD system signaling that contributes to fibrogenesis at the downstream of TGF-β, and arrived at completion of the present invention.

The present invention is as follows:
[1] A phosphorylation inhibitor of SMAD2/3 protein, comprising an antisense nucleic acid that suppresses NEK6 gene expression as an active ingredient;
[2] A therapeutic agent for fibrosis, comprising an antisense nucleic acid that suppresses NEK6 gene expression as an active ingredient;
[3] The therapeutic agent according to [2], wherein the fibrosis is pulmonary fibrosis, hepatic fibrosis, or kidney fibrosis;
[4] An antisense nucleic acid that suppresses NEK6 gene expression, comprising a sequence selected from the group consisting of a sequence having at least 90% sequence identity to a sequence of SEQ ID NO: 1, a sequence having at least 90% sequence identity to a sequence of SEQ ID NO: 2, and a sequence having at least 90% sequence identity to a sequence of SEQ ID NO: 3;
[5] An antisense nucleic acid that suppresses NEK6 gene expression, comprising a sequence selected from the group consisting of a sequence of SEQ ID NO: 1, a sequence of SEQ ID NO: 2, and a sequence of SEQ ID NO: 3;
[6] A method for inhibiting phosphorylation of SMAD2/3 protein, comprising administering an antisense nucleic acid that suppresses NEK6 gene expression to a subject;
[7] A method for treating fibrosis, comprising administering an antisense nucleic acid that suppresses NEK6 gene expression to a subject;
[8] An antisense nucleic acid that suppresses NEK6 gene expression for use in inhibiting the phosphorylation of SMAD2/3 protein;
[9] An antisense nucleic acid that suppresses NEK6 gene expression for use in treating fibrosis;
[10] Use of an antisense nucleic acid that suppresses NEK6 gene expression for producing a phosphorylation inhibitor of SMAD2/3 protein; and
[11] Use of an antisense nucleic acid that suppresses NEK6 gene expression for producing a therapeutic agent for fibrosis;
[12] The use according to [11], wherein the nucleic acid that suppresses NEK6 gene expression is a nucleic acid comprising a sequence of KB-XAX, KB-XBX or KB-XCX;
[13] A phosphorylation inhibitor of SMAD2/3 protein, comprising a nucleic acid that suppresses NEK6 gene expression as an active ingredient, wherein the nucleic acid targets a sequence on the 3' untranslated region of NEK6 gene mRNA;
[14] A therapeutic agent for fibrosis, comprising a nucleic acid that suppresses NEK6 gene expression as an active ingredient, wherein the nucleic acid targets a sequence on the 3' untranslated region of NEK6 gene mRNA;
[15] The therapeutic agent according to [14], wherein the fibrosis is pulmonary fibrosis, hepatic fibrosis, or kidney fibrosis;
[16] The inhibitor according to [13], wherein the nucleic acid that suppresses NEK6 gene expression comprises a sequence selected from the group consisting of a sequence having at least 90% sequence identity to a sequence of SEQ ID NO: 1, a sequence having at least 90% sequence identity to a sequence of SEQ ID NO: 2, and a sequence having at least 90% sequence identity to a sequence of SEQ ID NO: 3;
[17] The inhibitor according to [13], wherein the nucleic acid that suppresses NEK6 gene expression comprises a sequence selected from the group consisting of a sequence of SEQ ID NO: 1, a sequence of SEQ ID NO: 2, and a sequence of SEQ ID NO: 3;
[18] A method for inhibiting phosphorylation of SMAD2/3 protein, comprising administering to a subject a nucleic acid that suppresses NEK6 gene expression, wherein the nucleic acid targets a sequence on the 3' untranslated region of NEK6 gene mRNA;
[19] A method for treating fibrosis, comprising administering to a subject a nucleic acid that suppresses NEK6 gene expression, wherein the nucleic acid targets a sequence on the 3' untranslated region of NEK6 gene mRNA;
[20] A nucleic acid that suppresses NEK6 gene expression for use in inhibiting phosphorylation of SMAD2/3 protein, wherein the nucleic acid targets a sequence on the 3' untranslated region of NEK6 gene mRNA;
[21] A nucleic acid that suppresses NEK6 gene expression for use in treating fibrosis, wherein the nucleic acid targets a sequence on the 3' untranslated region of NEK6 gene mRNA;
[22] Use of a nucleic acid that suppresses NEK6 gene expression for producing a phosphorylation inhibitor of SMAD2/3 protein, wherein the nucleic acid targets a sequence on the 3' untranslated region of NEK6 gene mRNA;
[23] Use of a nucleic acid that suppresses NEK6 gene expression for producing a therapeutic agent for fibrosis, wherein the nucleic acid targets a sequence on the 3' untranslated region of NEK6 gene mRNA;
[24] The use according to [23], wherein the nucleic acid that suppresses NEK6 gene expression is a nucleic acid comprising a sequence of KB-XAX, KB-XBX or KB-XCX;
[25] The inhibitor according to [1], wherein the number of nucleotides contained in the antisense nucleic acid that suppresses NEK6 gene expression is 10- to 40-nt;
[26] The inhibitor according to [1], wherein the number of nucleotides contained in the antisense nucleic acid that suppresses NEK6 gene expression is 12- to 21-nt;
[27] The inhibitor according to [1], wherein an artificial nucleic acid is introduced at both ends of a sequence of the antisense nucleic acid that suppresses NEK6 gene expression;
[28] The inhibitor according to [1], wherein the number of nucleotides contained in the antisense nucleic acid that suppresses NEK6 gene expression is 12- to 21-nt, and an artificial nucleic acid is introduced at both ends of a sequence of the antisense nucleic acid;
[29] The therapeutic agent according to [2], wherein the number of nucleotides contained in the antisense nucleic acid that suppresses NEK6 gene expression is 10- to 40-nt;
[30] The therapeutic agent according to [2], wherein the number of nucleotides contained in the antisense nucleic acid that suppresses NEK6 gene expression is 12- to 21-nt;
[31] The therapeutic agent according to [2], wherein an artificial nucleic acid is introduced at both ends of a sequence of the antisense nucleic acid that suppresses NEK6 gene expression;
[32] The therapeutic agent according to [2], wherein the number of nucleotides contained in the antisense nucleic acid that suppresses NEK6 gene expression is 12- to 21-nt, and an artificial nucleic acid is introduced at both ends of a sequence of the antisense nucleic acid;
[33] The therapeutic agent according to [32], wherein the antisense nucleic acid is administered by being encapsulated in a liposome, glycolic acid copolymer (PLGA) microsphere, lipid microsphere, polymeric micelle, gelling agent, or exosome drug delivery carrier.

### Advantageous Effects of Invention

According to the present invention, a novel phosphorylation inhibitor of SMAD2/3 protein and a therapeutic agent for fibrosis can be provided.

### Brief Description of Drawings

[Figure 1] Figure 1 is a drawing showing a sequence of NEK6 gene mRNA and a target site of each antisense nucleic acid made in Example 1. The oblique line portions indicate untranslated regions. Note that U (uracil) on the mRNA sequence is denoted as T (thymine).
[Figure 2] Figure 2 is a graph showing the amounts of the transcripts of NEK6 gene when each antisense nucleic acid was introduced.
[Figure 3] Figure 3 represents results of Western blot for phosphorylated SMAD3 protein when NEK6 was knocked down by each antisense nucleic acid.
[Figure 4] Figure 4 shows results of quantifying the results of Western blot of phosphorylated SMAD3 protein when NEK6 was knocked down by each antisense nucleic acid, and calibrating the phosphorylated SMAD3 amount by the total SMAD3 amount.
[Figure 5] Figure 5a represents results of co-immunoprecipitation with an anti-NEK6 antibody. Figure 5b represents results of co-immunoprecipitation with an anti-FLAG antibody.
[Figure 6] Figure 6 represents results of Western blot for phosphorylated SMAD3 protein when His-fused NEK6 protein and GST-fused SMAD3 protein were reacted.
[Figure 7] Figure 7a represents results of Western blot when NEK6 was knocked down by siRNA. Figure 7b shows luminescence quantity of firefly luciferase when NEK6 was knocked down.
[Figure 8] Figure 8 shows the transcript amounts of Collal gene when NEK6 was knocked down by each antisense nucleic acid in hepatic stellate cells.
[Figure 9] Figure 9 shows the transcript amounts of Fibronectin gene when NEK6 was knocked down by each antisense nucleic acid in hepatic stellate cells.
[Figure 10] Figure 10a shows the transcript amounts of Collal gene when NEK6 was knocked down by siRNA in pulmonary fibroblasts.
Figure 10b shows the transcript amounts of αSMA gene when NEK6 was knocked down by siRNA in pulmonary fibroblasts.
[Figure 11] Figure 11 represents results of Western blotting for phosphorylated SMAD3 protein when NEK6 was knocked down by siRNA in kidney fibroblasts.
[Figure 12] Figure 12a represents measurement results of serum GPT when NEK6 was knockdown by siRNA in a CCl₄ models. Figure 12b represents measurement results of serum GOT when NEK6 was knockdown by siRNA in CCl₄ models.
[Figure 13] Figure 13 represents results of Western blotting for phosphorylated SMAD3 protein when NEK6 was knockdown by siRNA in CCl₄ models.
[Figure 14] Figure 14a shows the transcript amounts of NEK6 gene when NEK6 was knocked down by siRNA in CCl₄ models. Figure 14b shows the transcript amounts of Collal gene when NEK6 was knocked down by siRNA in CCl₄ models. Figure 14c shows the transcript amounts of Col3a1 gene when NEK6 gene was knockdown by siRNA in CCl₄ models. Figure 14d shows the transcript amounts of Timp1 gene when NEK6 gene was knockdown by siRNA in CCl₄ models.
[Figure 15] Figure 15a shows the transcript amounts of NEK6 gene when NEK6 was knockdown by siRNA in BDL models. Figure 15b shows the transcript amounts of Collal gene when NEK6 was knockdown by siRNA in BDL models. Figure 15c shows the transcript amounts of Col3a1 gene when NEK6 gene was knockdown by siRNA in BDL models. Figure 15d shows the transcript amounts of Timp1 gene when NEK6 gene was knockdown by siRNA in BDL models.
[Figure 16] Figure 16 represents results of pathological analysis of the liver when NEK6 was knockdown by siRNA in CCl₄ models. Figure 16a represents a result of the saline administration group not receiving CCl₄. Figure 16b represents a result of the solvent administration group receiving CCl₄. Figure 16c represents a result of the nucleic acid administration group receiving CCl₄.

### Description of Embodiments

The terms herein used are used to mean as commonly used in the art unless otherwise stated. Additionally, in the present invention, "number of nucleotides" means, for example, "length", and can also be referred to as "nucleotide length". In the present invention, the range of the number of nucleotides discloses, for example, all of the positive integers falling within the range, and as a specific example, the description "1- to 4-nt" means the disclosure of all of "1-, 2-, 3-, 4-nt".

The present invention provides a phosphorylation inhibitor of SMAD2/3 protein, comprising an antisense nucleic acid that suppresses NEK6 gene expression as an active ingredient, and a therapeutic agent for fibrosis, comprising the nucleic acid as an active ingredient. The present invention also provides a phosphorylation inhibitor of SMAD2/3 protein, comprising a nucleic acid that suppresses NEK6 gene expression, wherein the nucleic acid targets a sequence on the 3' untranslated region of NEK6 gene mRNA as an active ingredient, and a therapeutic agent for fibrosis, comprising the nucleic acid as an active ingredient. Hereinafter, the contents of the present invention will be described in detail.

### (1) Nucleic acids that suppresses NEK6 gene expression

NEK6 protein is one of 11 NIMA-related serine/threonine kinase families involving in control of cell division, and is phosphorylated (activated) in M-phase of cell cycle.

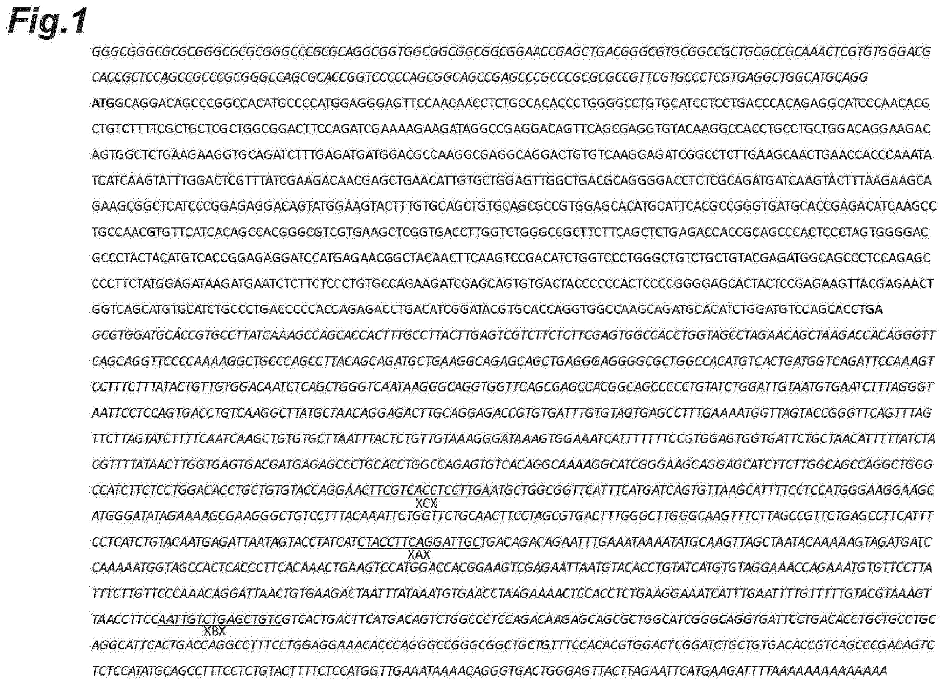

NEK6 gene, which is a target of the present invention, is a mammal-derived gene, and is preferably human-derived gene. Human-derived NEK6 gene has been reported for 7 variants, among which the mRNA sequence of Isoform 2 (accession number of GenBank of NCBI: NM_014397) is shown in Figure 1 (SEQ ID NO: 4).

The mechanism of suppressing NEK6 gene expression by a nucleic acid molecule is not particularly limited, and is simply required enabling the expression to be down regulated. Suppression of the expression of NEK6 gene can be confirmed by decrease in production of transcription product from NEK6 gene, decrease in production of translation product from NEK6 gene, or decrease in activity of the translation product.

The nucleic acids that suppress NEK6 gene expression include antisense nucleic acids, siRNAs, ssPN molecules, ssNc molecules, miRNAs, ribozymes, and the like of NEK6 mRNA. The nucleic acid may be a single-stranded nucleic acid or a double-strand nucleic acid.

The antisense nucleic acids, siRNAs, ssPN molecules, ssNc molecules, and ribozymes can be easily obtained by those skilled in the art on the basis of the nucleotide sequence of human NEK6 gene described above. A nucleic acid prepared on the basis of the mRNA sequence of NEK6 isoform 2 (SEQ ID NO: 4) is preferred. For example, the nucleic acid can be prepared on the basis of the 3' untranslated region on the mRNA sequence of NEK6 isoform 2.

### (2) Antisense Nucleic Acid

An antisense nucleic acid is an oligonucleotide (antisense DNA and/or antisense RNA) that forms a sequence-specific duplex with a target mRNA and performs functional inhibition such as splicing inhibition and translation inhibition thereof, and exerts an effect by introducing into a cell an antisense nucleic acid against the full length or a portion of a target mRNA.

Mechanisms of expression inhibition by an antisense nucleic acid include:
1) steric inhibition of a translation initiation complex by directing to a region from the 5' cap site of mRNA to about 25-nucleotide downstream of the initiation codon as a target sequence,
2) mRNA degradation via RNaseH with a single-strand DNA complementary to a target mRNA, and
3) splicing inhibition that directs to a boundary region between an exon and an intron of a pre-mRNA as a target sequence (mRNA maturation inhibition).

However, the mechanisms are not particularly limited as long as it suppresses NEK6 gene expression.

With regard to the sequence contained in the antisense nucleic acid according to the present embodiments, it is preferable to select a sequence specific to NEK6 gene. Examples include to select a sequence which is complementary to NEK6 gene and is not complementary to NEK7 gene (RefSeq database: NM_133494.2) or has one or more (e.g., 1 to 3) nucleotides which are uncomplimentary (mismatched) to NEK7 gene in the region. Increasing nucleotides (e.g., 4 or more, preferably 5 to 7 nucleotides) which are complementary to NEK6 gene and uncomplimentary (mismatched) to NEK7 gene is useful in avoiding off-target effect.

The number of nucleotides contained in the antisense nucleic acid according to the present embodiments is not particularly limited as long as the antisense nucleic acid exerts an effect of suppressing NEK6 gene expression, but may be, for example, 10- to 40-nt, 10- to 35-nt, 12-to 30-nt, or 12- to 21-nt.

The antisense nucleic acid that suppresses NEK6 gene expression can be obtained on the basis of cDNA sequence information of NEK6 mature mRNA, for example, according to an antisense nucleic acid sequence-designing system such as Antisense LNA(R) GapmeR design tool.

It is preferable that the antisense nucleic acid contain a modified nucleotide residue in view of binding stability with RNA (such as Tm value), mismatch sequence recognition capability, nuclease resistance, RNaseH activity, and the like.

Specific examples of the antisense nucleic acid that suppresses NEK6 gene expression include the following single-strand nucleic acid molecules.
[1] A nucleic acid containing a sequence having at least 90% sequence identity to a sequence of SEQ ID NO: 1.
[2] A nucleic acid containing a sequence having at least 90% sequence identity to a sequence of SEQ ID NO: 2.
[3] A nucleic acid containing a sequence having at least 90% sequence identity to a sequence of SEQ ID NO: 3.

The sequence identity of the sequence contained in the antisense nucleic acid to the sequence of SEQ ID NOs: 1 to 3 may be 90% or more, 92% or more, 95% or more, or 98% or more, or 100%. Various modifications may be added to the antisense nucleic acids [1] to [3] to the extent that the effect of suppressing NEK6 gene expression is not lost. The various modifications may be present within the sequence of SEQ ID NOs: 1 to 3, or may be present outside the sequence. Details of the modified nucleotides and the like are described later.

The antisense nucleic acid that suppresses NEK6 gene expression can be easily obtained on the basis of the nucleotide sequence of NEK6 gene by known methods. For example, it can be designed appropriately on the basis of the mRNA sequence information of NEK6 gene such as the sequence of mRNA of NEK6 isoform 2 (SEQ ID NO: 4), and synthesized by a method known in the art. Examples of the synthesis methods include a synthesis method by a genetic engineering technique, and a chemical synthesis method. Examples of genetic engineering techniques include an in vitro transcription synthesis method, a method using a vector, and a method by a PCR cassette. The vector is not particularly limited, and examples include non-viral vectors such as plasmids, and viral vectors. The chemical synthesis method is not particularly limited, and examples include a phosphoroamidite method and an H-phosphonate method. The chemical synthesis method can utilize, for example, a commercially-available automated nucleic acid synthesizer.

### (3) siRNAs

An siRNA (small interfering RNA), one of nucleic acids that suppress NEK6 gene expression, will be described below.

An siRNA is a nucleic acid molecule that consists of a guide strand (antisense strand) to pair with a target gene, and a passenger strand (sense strand) forming a double strand together with the guide strand. Within a cell, an siRNA is incorporated into a complex referred to as RNA-inducing silencing complex (RISC) that involves Argonaute (AGO) protein as a central core, and then the sense strand is degraded by AGO and the guide strand remains in RISC. A seed region in a guide strand (a 7-nt region at positions 2 to 8 from the 5' end of the guide strand) has been considered to have an important function in recognizing a target sequence, and it has been believed to be preferable to select a seed region specific to a target gene for the purpose of avoiding off-target effect. Accordingly, with regard to the seed region of the nucleic acid that is an active ingredient of the present invention, it is also preferable to select a sequence specific to NEK6 mature mRNA (RefSeq database: NM 014397). Such examples include selecting a nucleic acid containing, as the seed region, a sequence which is complementary to NEK6 mature mRNA and is not complementary to NEK7 mature mRNA (RefSeq database NM_133494.2) or in which one or more (e.g., 1 to 3) nucleotides in the region is uncomplimentary (mismatched) to NEK7 mature mRNA. Furthermore, also with regard to the full length sequence, it is useful, for example, to increase nucleotides which is complementary to NEK6 mature mRNA and uncomplimentary (mismatched) to NEK7 mature mRNA (e.g., 4 or more, preferably 5 to 7 nucleotides), for the purpose of avoiding off-target effect. The number of nucleotides in a sequence suppressing expression contained in a guide strand is, for example, 15 to 30, preferably 19 to 25, more preferably 19 to 23, yet preferably 21, 22, 23, and particularly preferably 23.

The sequence suppressing expression described above may further have an additional sequence at the 3' side to form an overhanging end. The number of nucleotides in the additional sequence described above is, for example, 1 to 11, and preferably 1 to 4. The additional sequence may be ribonucleotide residues or deoxyribonucleotide residues.

The number of nucleotides in the guide strand is, for example, 19- to 50-nt, preferably 19- to 30-nt, more preferably 19- to 25-nt, yet preferably 19- to 23-nt, yet more preferably 21-, 22-, 23-, and particularly preferably 23-nt.

The number of nucleotides in the passenger strand is, for example, 19- to 50-nt, preferably 19- to 30-nt, more preferably 19- to 25-nt, yet preferably 19- to 23-nt, yet more preferably 21-, 22-, 23-nt, and particularly preferably 21-nt.

In the passenger strand, a region showing complementarity to the guide strand is, for example, 19- to 50-nt, preferably 19- to 30-nt, more preferably 19- to 25-nt, and yet preferably 19- to 23-nt in length. The region may further have an additional sequence at the 3' side. The number of nucleotides in the additional sequence is, for example, 1- to 11-nt, and preferably 1- to 4-nt, and the additional sequence may be of ribonucleotide residues or deoxyribonucleotide residues. The passenger strand, for example, may be complementary to the region showing complementarity to the guide strand, or may have one or several nucleotides which are uncomplimentary, but it is preferable to be complementary. The one nucleotide or several nucleotides means, for example, 1- to 3-nt, and preferably 1-nt or 2-nt.

An siRNA that suppresses NEK6 gene expression can be obtained on the basis of cDNA sequence information of NEK6 mature mRNA, for example, according to a siRNA-designing system such as siSNIPER(R), or siDirect(R) for drug discovery/diagnostic research.

It is preferable NEK6 siRNA be an siRNA that specifically acts on NEK6, and examples include double-strand nucleic acids as follows:
(a) a double-strand nucleic acid molecule comprising a guide strand (antisense strand) containing a sequence having at least 90% sequence identity to a sequence of SEQ ID NO: 1, and a passenger strand (sense strand)
(b) a double-strand nucleic acid molecule comprising a guide strand (antisense strand) containing a sequence having at least 90% sequence identity to a sequence of SEQ ID NO: 2, and a passenger strand (sense strand)
(c) a double-strand nucleic acid molecule comprising a guide strand (antisense strand) containing a sequence having at least 90% sequence identity to a sequence of SEQ ID NO: 3, and a passenger strand (sense strand)

The sequence identity of the sequence contained in the guide strand (antisense strand) comprised in the double-strand nucleic acid molecule to the sequence of SEQ ID NOs: 1 to 3 may be 90% or more, 92% or more, 95% or more, or 98% or more, or 100%.

The siRNA that suppresses NEK6 gene expression may also be a single-strand nucleic acid molecule forming a hairpin RNA structure, wherein the 3' end of the passenger strand (sense strand) and the 5' end of the guide strand (antisense strand) are linked to each other via a linker sequence of a nucleotide residue and/or a linker of a non-nucleotide structure, or the 3' end of the guide strand (antisense strand) and the 5' end of the passenger strand (sense strand) are linked to each other via a linker sequence of a nucleotide residue and/or a linker of a non-nucleotide structure.

### (4) ssPN molecules

An ssPN molecule to be one of the nucleic acid that suppresses NEK6 gene expression will be described. An ssPN molecule means a single-strand RNA nucleic acid molecule having excellent biological stability, which is disclosed in WO2012/017919, and is particularly as follows.

The ssPN molecule as an active ingredient of the present invention is a single-strand nucleic acid molecule containing a sequence suppressing NEK6 gene expression, and is characterized by containing a region (X), a linker region (Lx), and a region (Xc); wherein the linker region (Lx) is linked between the region (X) and the region (Xc); wherein at least one of the region (X) and the region (Xc) contains the sequence suppressing the expression; wherein the linker region (Lx) has a non-nucleotide structure containing at least one of a pyrrolidine skeleton and a piperidine skeleton. The ssPN molecule has the 5' end and the 3' end unlinked, and can also be referred to as a linear single-strand nucleic acid molecule.

In the ssPN molecule, a sequence suppressing NEK6 gene expression is, for example, a sequence that exhibits a suppressing activity on NEK6 gene expression when the ssPN molecule of the present invention is introduced into a cell *in vivo* or *in vitro.* An siRNA sequence to bind to NEK6 mRNA can be obtained in accordance with an existing siRNA-designing system on the basis of cDNA sequence information of NEK6 gene, and the ssPN molecule can also employ the sequence suppressing expression for siRNA as a sequence suppressing expression for the ssPN molecule.

It is preferable that the sequence suppressing expression have, for example, an 80% or more of complementarity to a target region of NEK6 gene, which is more preferably 90% or more, yet preferably 95% or more, yet more preferably 98% or more, and particularly preferably 100%.

In particular, with regard to a part corresponding to a seed region of siRNA, it is preferable to select a sequence specific to NEK6 gene as similar to the case of siRNA.

Suppression of NEK6 gene expression caused by the ssPN molecule is estimated to be due to, for example, occurrence of RNA interference, but is not limited by this mechanism. The ssPN molecule of the present invention is not one which is introduced into a cell or the like as a dsRNA consisting of two single-strand RNAs, such as so-called siRNA, and furthermore, excision of the sequence suppressing the expression is not necessarily essential within a cell.

In the ssPN molecule, the linker region (Lx) may have, for example, a non-nucleotide structure containing the pyrrolidine skeleton, or may have a non-nucleotide structure containing the piperidine skeleton, or may have both of a non-nucleotide structure containing the pyrrolidine skeleton and a non-nucleotide structure containing the piperidine skeleton.

### (5) ssNc molecule

An ssNc molecule which is one of nucleic acids that suppress NEK6 gene expression will be described.

An ssNc molecule means a single-strand RNA nucleic acid molecule disclosed in WO2012/05368, and is specifically as follows. The ssNc molecule is a single-strand nucleic acid molecule containing a sequence suppressing expression that suppresses the expression of a target gene, and
contains a 5' side region (Xc), an inner region (Z), and a 3' side region (Yc) in this order from the 5' side to the 3' side,
wherein the inner region (Z) consists of an inner 5' side region (X) linked to an inner 3' side region (Y),
wherein the 5' side region (Xc) is complementary to the inner 5' side region (X),
wherein the 3' side region (Yc) is complementary to the inner 3' side region (Y),
wherein at least one of the inner region (Z), the 5' side region (Xc), and the 3' side region (Yc) contains the sequence suppressing expression.

The ssNc molecule has the 5' end and 3' end unlinked, and can also be referred to as a linear single-strand nucleic acid molecule. The ssNc molecule of the present invention, for example, has the inner region (Z) in which the inner 5' region (X) and the inner 3' region (Y) are directly linked.

In ssNc molecule, the 5' side region (Xc) is complementary to the inner 5' side region (X), and the 3' side region (Yc) is complementary to the inner 3' side region (Y). Hence, in the 5' side, the region (Xc) folds toward the region (X), and the region (Xc) and the region (X) can form a duplex strand through self-annealing, whereas on the 3' side, the region (Yc) folds toward the region (Y), and the region (Yc) and the region (Y) can form a duplex strand through self-annealing.

The ssNc molecule, thus, can form a duplex strand within a molecule, and has a structure clearly different from that in which two separate single-strand RNAs form a double-strand RNA thorough annealing, for example, as siRNAs used for a conventional RNA interference.

The sequence suppressing expression in the ssNc molecule can employ the description for ssPN molecules.

Suppression of the expression of NEK6 gene by the ssNc molecule is estimated to be caused by, for example, taking a structure in which the sequence suppressing expression is disposed in at least one of the inner region (Z), the 5' side region (Xc), and the 3' side region (Yc), thereby leading to RNA interference or a phenomenon similar to RNA interference (RNA interference-like phenomenon). Here, mechanisms of the ssNc molecules are also not limited, as are the cases with mechanisms of the ssPN molecules. The ssNc molecule is not one that is introduced into a cell or the like as a dsRNA consisting of two single-strand RNAs, such as so-called siRNA, and furthermore, excision of the sequence suppressing expression is not necessarily essential within a cell. Thus, ssNc molecules can also be considered to have, for example, RNA interference-like function.

### (6) Synthesis method of ssPN molecule and ssNc molecule

Synthesis methods of ssPN molecules and ssNc molecules are not particularly limited, and can employ conventional known methods. Examples of the synthesis methods include a synthesis method by a genetic engineering technique, and a chemical synthesis method. Examples of genetic engineering techniques include an *in vitro* transcription synthesis method, a method using a vector, and a method by a PCR cassette. The vector is not particularly limited, and examples include non-viral vectors such as plasmids, and viral vectors. The chemical synthesis method is not particularly limited, and examples include a phosphoroamidite method and an H-phosphonate method. The chemical synthesis method can utilize, for example, a commercially-available automated nucleic acid synthesizer. In the chemical synthesis method, amidite is generally used. The amidite is not particularly limited, and examples of commercially-available amidites include RNA Phosphoramidites (2'-O-TBDMSi, trade name, Samchully Pharmaceutical Co., Ltd), ACE amidite, TOM amidite, CEE amidite, CEM amidite, and TEM amidite. Moreover, the ssPN molecule and ssNc molecule of the present invention can be manufactured according to manufacture methods described in WO2012/05368, WO2012/17919, WO2013/27843, and WO2016/159374.

### (7) miRNA

An miRNA which is one of nucleic acids that suppress NEK6 gene expression will be described below.

An miRNA participates in regulation of gene expression through inhibition of translation from mRNA to protein or degradation of mRNA. An miRNA is a short-strand (20- to 25-nt) non-coding RNA present within a cell. At first, an miRNA is transcribed as a single-strand pri-RNA that contains an miRNA and the complementary strand thereof and can take a hairpin loop structure from DNA. Next, the pri-RNA is cut out with a portion by an enzyme called Drosha within a nucleus, converted to a pre-RNA, and transported outside the nucleus. Then, the pre-RNA is further cleaved by Dicer, thereby functioning as an miRNA. The miRNA executes incomplete hybridization binding to the 3' untranslated region of mRNA to inhibit synthesis of protein encoded by the mRNA.

The miRNA that suppresses NEK6 gene expression can be obtained on the basis of gene name or mRNA sequence information of a target gene, for example, according to a database such as miRDB (http://mirdb.org/miRDB/index.html).

### (8) Nucleotide residues used for nucleic acid

The nucleotide residue used for a nucleic acid that is an active ingredient in the present embodiments contains a sugar, a base, and phosphate as components. Examples of the nucleotide residues include ribonucleotide residues and deoxyribonucleotide residues. The ribonucleotide residue, for example, has a ribose residue as a sugar, and has adenine (A), guanine (G), cytosine (C), and uracil (U) as a base; and the deoxyribose residue, for example, has a deoxyribose residue as a sugar, and has adenine (A), guanine (G), cytosine (C), and thymine (T) as a base.

The nucleotide residues include unmodified nucleotide residues and modified nucleotide residues. In the unmodified nucleotide residue, each of the components is, for example, identical or substantially identical to naturally occurring one, and preferably identical or substantially identical to naturally occurring one in human body.

The modified nucleotide residue is, for example, a nucleotide residue in which the unmodified nucleotide residue is modified. In the modified nucleotide, for example, any of components in the unmodified nucleotide residue may be modified. In the present invention, "modification" represents, for example, substitution, addition, and/or deletion of the component, and substitution, addition, and/or deletion of an atom and/or a functional group in the component, and can be referred to as "alteration". Examples of the modified nucleotide residues include a naturally occurring nucleotide residue, and an artificially modified nucleotide residue. The naturally-originated modified nucleotide residue can refer to, for example, Limbach et al. (Limbach et al., 1994, Summary: the modified nucleosides of RNA, Nucleic Acids Res., 22:2183-2196). Additionally, the modified nucleotide residue may be, for example, an alternative residue of the nucleotide.

Examples of modifications of the nucleotide residues include modification of a ribose-phosphate skeleton (hereinafter referred to as a ribophosphate skeleton). In the ribophosphate skeleton, for example, a ribose residue can be modified. The ribose residue can be, for example, modified at carbon of position 2', and can be specifically, for example, substituted at a hydroxyl group bound to carbon 2' by hydrogen or fluoro. By substituting a hydroxyl group on the carbon 2' by hydrogen, a ribose residue can be substituted with deoxyribose. It also can be modified by introducing a methyl group or a methoxyethyl group into the hydroxyl group at position 2', and examples thereof include 2'-F, 2'-O-Methyl (2'-OMe), and 2'-O-Methoxyethyl (2'-MOE). The ribose residue can be, for example, substituted with a stereoisomer, and may be, for example, substituted with an arabinose residue.

The ribophosphate skeleton may be substituted, for example, a non-ribophosphate skeleton having a non-ribose residue and/or non-phosphate. Examples of the non-ribophosphate skeletons include an uncharged form of the ribophosphate skeleton. Examples of the nucleotide alternatives having substitution with the non-ribophosphate skeleton include morpholino, cyclobutyl, and pyrrolidine. In addition to these, examples of the alternatives include artificial nucleic acid monomer residues. Specific examples thereof include peptide nucleic acid (PNA), and crosslinked artificial nucleic acids such as 2',4'-bridged nucleic acid [BNA, also known as locked nucleic acid (LNA)], AmNA, 2'-O,4'-C-ethylenebridged nucleic acids (ENA), GuNA, and scpBNA. When the nucleic acid according to the present embodiments is an antisense nucleic acid utilizing the mechanism of RNA degradation by RNaseH, it is preferable that the nucleic acid is an antisense nucleic acid in which an artificial nucleic acid is introduced, further preferably a nucleic acid in which artificial nucleic acids are introduced at both ends of a sequence of the antisense nucleic acid, and particularly preferably an antisense nucleic acid in which artificial nucleic acids are introduced only at both ends of the sequence of the antisense nucleic acid. This is because, by using an antisense nucleic acid in which an artificial nucleic acid is introduced only at both ends of the sequence of the antisense nucleic acid, RNA degradation by RNaseH occurs while the characteristics of the artificial nucleic acid are maintained, and a high antisense effect can be expected.

Meanwhile, when the nucleic acid according to the present embodiments is an antisense nucleic acid utilizing an exon skipping induction mechanism, it is preferable that the nucleic acid have a design avoiding RNA degradation by RNaseH, such as a mixmer or a total modified oligonucleotide, in order to prevent cleavage of a target RNA.

In the ribophosphate skeleton, for example, a phosphate group can be modified. In the ribophosphate skeleton, a phosphate group closest to a sugar residue is referred to as α phosphate group. The α phosphate group is negatively charged, and the electric charges are uniformly distributed over two oxygen atoms unbound to the sugar residue. Among four oxygen atoms in the α phosphate group, two oxygen atoms unbound to the sugar residue in a phosphodiester bond between nucleotide residues is also hereinafter referred to as "unbinding (non-linking) oxygen". In contrast, two oxygen atoms bound to the sugar residue in the phosphodiester bond between the nucleotide residues is hereinafter referred to as "binding (linking) oxygen". It is preferable that the α phosphate group be subjected to, for example, modification to undergo uncharging, or modification to allow the electric charge distribution on the unbinding atom to be an asymmetry type.

The phosphate group may be substituted, for example, at the unbinding oxygen. The oxygen can be, for example, substituted with any atom of S (sulfur), Se (selenium), B (boron), C (carbon), H (hydrogen), N (nitrogen), and OR (R is, for example, an alkyl group or an aryl group), and preferably substituted with S. It is preferable that in the unbinding oxygens, for example, both be substituted, and more preferably, both are substituted with S. Examples of the modified phosphate groups include phosphorothioate, phosphorodithioate, phosphoroselenate, boranophosphate, boranophosphate ester, phosphonatehydrogen, phosphoramidate, alkyl or arylphosphonate, and phosphotriester, and among them, phosphorodithioate in which both of the two unbinding oxygens are substituted with S is preferable.

The phosphate group may be substituted, for example, at the binding oxygen. The oxygen can be substituted with, for example, any atom of S (sulfur), C (carbon), and N (nitrogen), or borane (BH₃). Examples of the modified phosphate groups include a cross-linking phosphoroamidate having substitution with N, a cross-linking phosphorothioate having substitution with S, and a cross-linking methylenephosphonate having substitution with C. The phosphorothioate modification (sulfuration) in which the oxygen atom is substituted with an S (sulfur) atom is a modification of a phosphodiester linkage connecting a nucleic acid with a nucleic acid, and alters the phosphodiester linkage in the sequence to a phosphorothioate linkage. This is preferred because it can be expected to improve nuclease resistance. The substitution of the binding oxygen may be made, for example, on at least one of the 5' end nucleotide residue and the 3' end nucleotide residue, or on both of the nucleotide residues in the nucleic acid according to the present embodiments. Furthermore, for example, all phosphate diester linkages in the nucleic acid according to the present embodiments may be altered to phosphorothioate linkages.

The phosphate group may be substituted with, for example, the phosphorous-free linker described above. The linkers include, for example, siloxane, carbonate, carboxymethyl, carbamate, amide, thioether, ethyleneoxide linker, sulfonate, sulfonamide, thioformacetal, formacetal, oxime, methyleneimino, methylenemethylimino, methylenehydrazo, methylenedimethylhydrazo, and methyleneoxymethylimino, and preferably include a methylenecarbonylamino group and a methylenemethylimino group.

Examples of modifications of the end nucleotide residue include addition of another molecule. Examples of the other molecules include functional molecules such as a labelling substance and a protecting group as mentioned above. Examples of the protecting groups include S (sulfur), Si (silicon), B (boron), and an ester-containing group.

The other molecule, for example, may be added to a phosphate group of the nucleotide residue, or may be added to the phosphate group or the sugar residue via a spacer. An end atom of the spacer may be added to or substituted with, for example, the binding oxygen of the phosphate group, or O, N, S, or C of the sugar residue. It is preferable that the binding site of the sugar residue be, for example, C of position 3' or C of position 5', or an atom bound thereto. The spacer can also be added to or substituted with, for example, an end atom of the nucleotide alternative such as the PNA.

The spacer is not particularly limited, and may contain, for example, -(CH₂)ₙ-, -(CH₂)ₙN-, -(CH₂)ₙO-, -(CH₂)ₙS-, O(CH₂CH₂O)ₙCH₂CH₂OH, non-base sugar, amide, carboxy, amine, oxyamine, oxyimine, thioether, disulfide, thiourea, sulfonamide, morpholino, and a biotin reagent, a fluorescein reagent. In the formula, n is a positive integer, and n = 3 or 6 is preferable.

In addition to these, examples of the molecules to be added to the end include dyes, intercalators (e.g., acridine), cross-linkers (e.g., psoralen, mitomycin C), porphyrin (TPPC4, texaphyrin, sapphyrin), polycyclic aromatic hydrocarbon (e.g., fenadine, dihydrofenadine), artificial endonucleases (e.g., EDTA), lipophilic carriers (e.g., cholesterol, cholic acid, adamantaneacetic acid, 1-pyrenebutyric acid, dihydrotestosteron, 1,3-bis-O(hexadecyl)glycerol, a geranyloxyhexyl group, hexadecylglycerol, borneol, menthol, 1,3-propanediol, a heptadecyl group, palmitic acid, myristic acid, O3-(oleoyl)lithocholic acid, O3-(oleoyl)cholic acid, dimethoxytrityl, or phenoxazine) and peptide complexes (e.g., Antennapedia peptides, Tat peptides), alkylators, phosphate, amino, mercapto, PEG (e.g., PEG-40K), MPEG, [MPEG]₂, polyamino, alkyl, substituted alkyl, radiolabeled markers, enzymes, haptens (e.g., biotin), transport/absorption accelerators (e.g., aspirin, vitamin E, folic acid), and synthetic ribonucleases (e.g., imidazole, bisimidazole, histamine, imidazole clusters, acridine-imidazole complexes, tetraaza macro-cyclic Eu³⁺ complexes).

The nucleic acid molecule may have modification of the 5' end with, for example, a phosphate group or a phosphate group analogue. Examples of the phosphate group include 5' monophosphate ((HO)₂(O)P-O-5'), 5' diphosphate ((HO)₂(O)P-O-P(HO)(O)-O-5'), 5' triphosphate ((HO)₂(O)P-O-(HO)(O)P-O-P(HO)(O)-O-5'), 5'-guanosine caps (7-methylated or unmethylated, 7m-G-O-5'-(HO)(O)P-O-(HO)(O)P-O-P(HO)(O)-O-5'), 5'-adenosine caps (Appp), any modified or unmodified nucleotide cap structure (N-O-5'-(HO)(O)P-O-(HO)(O)P-O-P(HO)(O)-O-5'), 5'-thiophospate (phosphorothioate: (HO)₂(S)P-O-5'), 5'-dithiophosphate (phosphorodithioate: (HO)(HS)(S)P-O-5'), 5'-phosphorothiolic acid ((HO)₂(O)P-S-5'), sulfur-substituted monophosphate, diphosphate, and triphosphate (such as 5'-α-thiotriphosphate or 5'-γ-thiotriphosphate), 5'-phosphoramidate ((HO)₂(O)P-NH-5', (HO)(NH₂)(O)P-O-5'), 5'-alkylphosphonate (e.g., RP(OH)(O)-O-5', (OH)₂(O)P-5'-CH₂, wherein R is alkyl [such as methyl, ethyl, isopropyl, or propyl]), and 5'-alkyletherphosphate (e.g., RP(OH)(O)-O-5', wherein R is alkylether [such as methoxymethyl or ethoxymethyl]).

In the nucleotide residue, the base is not particularly limited. The base may be, for example, a natural base or an unnatural base. The base may be, for example, naturally-originated or a synthesized one. The base can employ, for example, a common base, or a modified analogue thereof.

Examples of the bases include purine bases such as adenine and guanine, and pyrimidine bases such as cytosine, uracil, and thymine. The bases otherwise include inosine, thymine, xantine, hypoxantine, nubularine, isoguanisine, and tubercidine. Examples of the bases include alkyl derivatives such as 2-amino adenine, 6-methylated purine; alkyl derivatives such as 2-propylated purine; 5-halouracil and 5-halocytosine; 5-propynyluracil and 5-propynylcytosine; 6-azouracil, 6-azocytosine, and 6-azothymine; 5-uracil (pseudouracil), 4-thiouracil, 5-halouracil, 5-(2-aminopropyl)uracil, 5-aminoallyl uracil; 8-haloate, aminated, thiolated, thioalkylated, hydroxylated, and other 8-substituted purines; 5-trifluoromethylated and other 5-substituted pyrimidines; 7-methylguanine; 5-substituted pyrimidine; 6-azapyrimidine; N-2, N-6, and O-6 substituted purine (including 2-amino propyladenine); 5-propynyluracil and 5-propynylcytosine; dihydrouracil; 3-deaza-5-azacytosine; 2-amino purine; 5-alkyl uracil; 7-alkyl guanine; 5-alkyl cytosine; 7-deazaadenine; N6,N6-dimethyladenine; 2,6-diaminopurine; 5-amino-allyl-uracil; N3-methyluracil; substituted 1,2,4-triazole; 2-pyridinone; 5-nitroindole; 3-nitropyrrole; 5-methoxyuracil; uracil-5-oxyacetic acid; 5-methoxycarbonylmethyluracil; 5-methyl-2-thiouracil; 5-methoxycarbonylmethyl-2-thiouracil; 5-methylaminomethyl-2-thiouracil; 3-(3-amino-3-carboxypropyl)uracil; 3-methylcytosine; 5-methylcytosine; N⁴-acetylcytosine; 2-thiocytosine; N6-methyladenine; N6-isopentyladenine; 2-methylthio-N6-isopentenyladenine; N-methylguanine; and O-alkylate bases. Furthermore, examples of purines and pyrimidines include those disclosed in U.S. Patent No. 3,687,808, "Concise Encyclopedia Of Polymer Science And Engineering", p. 858-859, ed. Kroschwitz J.I., John Wiley & Sons, 1990, and Englisch et al., Angewandte Chemie, International Edition, 1991, 30, p.613.

### (9) Definition of other terms

The terms used in descriptions for the nucleic acid that is an active ingredient, the linker, and the like in the present embodiments are those commonly used in the art, and for example, can be represented as follows.

In the present invention, "alkyl" includes, for example, linear or branched alkyl groups. The number of carbons of the alkyl is not particularly limited, but is, for example, 1 to 30, and preferably 1 to 6 or 1 to 4. Examples of the alkyl groups include methyl, ethyl, n-propyl, isopropyl, n-butyl, isobutyl, sec-butyl, tert-butyl, n-pentyl, isopentyl, neopentyl, n-hexyl, isohexyl, n-heptyl, n-octyl, n-nonyl, n-decyl. Preferably, examples include methyl, ethyl, n-propyl, isopropyl, n-butyl, isobutyl, sec-butyl, tert-butyl, n-pentyl, isopentyl, neopentyl, n-hexyl, and isohexyl.

In the present invention, "aryl" includes, for example, monocyclic aromatic hydrocarbon groups and polycyclic aromatic hydrocarbon groups. Examples of the monocyclic aromatic hydrocarbon groups include phenyl. Examples of the polycyclic aromatic hydrocarbon groups include 1-naphthyl, 2-naphthyl, 1-anthryl, 2-anthryl, 9-anthryl, 1-phenanthryl, 2-phenanthryl, 3-phenanthryl, 4-phenanthryl, and 9-phenanthryl. Examples preferably include phenyl, and naphthyl such as 1-naphthyl and 2-naphthyl.

### (10) Phosphorylation inhibitor of SMAD2/3 protein

Inhibition of the phosphorylation of SMAD2/3 protein means that the phosphorylation of SMAD2 and/or SMAD3 promoted by TGF-β stimulation is inhibited (controlled).

SMAD2/3 is one of R-SMADs (receptor regulated SMADs) that undergo phosphorylation by TGF-β type I receptor, and after stimulation by TGF-β, phosphorylated (activated) SMAD2 and SMAD3 immigrate together with SMAD4, which is Co-SMAD (common partner SMAD), into the nucleus. A shown in Example 4, the inventors found that NEK6 protein interacts with SMAD2/3 protein within a cell and promotes the phosphorylation of SMAD2/3 protein. The phosphorylated SMAD2/3 protein forms a SMAD protein complex, immigrates into the nucleus, and enhances transcription of α-SMA, α2-collagen, interferon β, interleukine-5, VEGF, and the like.

Accordingly, inhibition of SMAD signal system by the phosphorylation inhibitor of SMAD2/3 protein according to the present embodiments enables transcriptional control of α-SMA, α2-collagen, interferon β, and the like, and is, in turn, useful for suppressing differentiation of a fibroblast, a hepatic stellate cell, or the like into a myofibroblast, controlling matrix synthesis caused by fibroblasts or the like, and regulating inflammatory and immune reactions, and the like, in a wound healing process.

### (11) Therapeutic agent for fibrosis

The therapeutic agent for fibrosis according to the present embodiments is a therapeutic agent for hepatic fibrosis, hepatic cirrhosis, viral hepatitis, autoimmune hepatitis, primary biliary hepatitis, nonalcoholic steatohepatitis, alcoholic liver disease, primary sclerosing cholangitis, hemochromatosis, Wilson's disease, α1-antitrypsin deficiency, non-viral congestive hepatic cirrhosis, drug-induced hepatic disorder, pancreatitis, pancreatic fibrosis, retinal fibrosis, vocal fold scarring, vocal cord mucosal fibrosis, laryngeal fibrosis, pulmonary fibrosis, pneumonitis, idiopathic pulmonary fibrosis, non-specific pneumonitis, idiopathic organizing pneumonia, desquamative pneumonitis, respiratory bronchiolitis-associated pneumonitis, acute pneumonitis, lymphocytic pneumonitis, sarcoidosis, chronic eosinophilic pneumonia, acute eosinophilic pneumonia, lymphangioleiomyomatosis, pulmonary alveolar proteinosis, Hermansky-Pudlak syndrome, pulmonary Langerhans cell histiocytosis, siderosis, amyloidosis, pulmonary alveolar microlithiasis, hypersensitivity pneumonitis, pneumoconiosis, infectious pulmonary disease, drug-induced pneumonia, radiation pneumonia, cystic fibrosis, myelofibrosis, kidney fibrosis, chronic renal failure, diabetic nephropathy, chronic glomerulonephritis, malignant nephrosclerosis, polycystic kidney, drug-induced renal disorder, retroperitoneal fibrosis, collagenosis, scleroderma, congenital dyskeratosis, nephrogenic systemic fibrosis, and additionally, diseases widely associated with fibrogenesis including airway fibrogenesis, intestinal fibrogenesis, urinary bladder fibrogenesis, prostatic fibrogenesis, and dermal fibrogenesis. Preferably, the fibrosis is hepatic fibrosis, hepatic cirrhosis, pulmonary fibrosis, pneumonitis, kidney fibrosis, or chronic renal failure, and more preferably pulmonary fibrosis, hepatic fibrosis, or kidney fibrosis.

An administration method of the therapeutic agent for fibrosis according to the present embodiments is not particularly limited, but it is preferable that it be parenteral administration such as inhalation, intravenous administration (intravenous injection), intramuscular administration, subcutaneous administration, intradermal administration, intrathecal administration, and transdermal administration. It is preferable that the therapeutic agent for fibrosis according to the present embodiments is administered to a subject in need of the therapeutic agent for fibrosis, for example, a subject having a disease related to fibrogenesis as described above and a subject determined to be at high risk for developing the disease. The subject may be a human or a non-human animal. The dosage of the nucleic acid molecule according to the present embodiments in a therapeutic method according to the present embodiments is not particularly limited as long as it is an effective amount for treating the disease described above, and varies depending on the type of disease, the degree of severity, age, body weight, route of administration, and the like, but may be typically about 0.0001 to about 100 mg/kg by body weight, for example, about 0.001 to about 10 mg/kg by body weight, and preferably about 0.005 to about 5 mg/kg by body weight, per once for an adult. Such amount can be administered at an interval of, for example, three times a day to once a month, preferably once a day to a week.

The therapeutic agent for fibrosis according to the present embodiments is typically formulated as an appropriate pharmaceutical composition with a pharmaceutically acceptable carrier, and administered in an oral or parenteral form.

The antisense nucleic acid according to the present embodiments is preferably administered by being encapsulated in a liposome, glycolic acid copolymer (PLGA) microsphere, lipid microsphere, polymeric micelle, gelling agent, or exosome drug delivery carrier.

Hereinafter, the present invention will be described in detail with Examples and the like, but the present invention is not limited to these. It should be noted that the "antisense oligonucleotide" has the same meaning as the "antisense nucleic acid" and "ASO" in the Examples.

### Examples

### [Example 1: Knockdown of NEK6 using antisense oligonucleotides]

Human NEK6 antisense oligonucleotides (KB-XAX, KB-XBX, KB-XCX) were transfected into human hepatic stellate cell line LI-90 cells using Lipofectamine RNAi MAX (Invitrogen(TM)) at a final concentration of 30 nM. 48 hours after transfection, the medium was changed from DMEM Low Glucose medium containing 10% FCS (Thermo Fisher Scientific, Inc.) to DMEM Low Glucose medium containing 0.2% FCS (Thermo Fisher Scientific, Inc.). 96 hours after transfection, RNAs were extracted from the cells transfected with the antisense oligonucleotides, using RNeasy Mini Kit (Qiagen N.V.).

As the antisense oligonucleotide sequences, the following KB-XAX, XBX and XCX were used.
NEK6 antisense oligonucleotides (KB-XAX): 5'-GCAATCCTGAAGGTAG-3' (SEQ ID NO: 1)
NEK6 antisense oligonucleotides (KB-XBX): 5'-GACAGCTCAGACAATT-3' (SEQ ID NO: 2)
NEK6 antisense oligonucleotides (KB-XCX): 5'-TCAAGGAGGTGACGAA-3' (SEQ ID NO: 3)

The RNAs thus obtained were subjected to reverse transcription using High Capacity cDNA Reverse Transcription Kit (Applied Biosystems(R)) to obtain cDNAs. The cDNAs thus obtained were subjected to real-time PCR using TaqMan Gene Expression Assays (Applied Biosystems(R)) to examine influence on the transcript amount of NEK6 gene by NEK6 knockdown. The transcript amount of NEK6 gene was calculated by dividing a measurement value in NEK6 Taqman Probe (HS00205221_m1, Applied Biosystems(R)) by a measurement value in 18s Probe. The following custom synthesized 18s MGB Probe as the 18s Probe, custom synthesized 18s Primer 1, and custom synthesized 18s Primer 2 were mixed so as to be 0.2 µM, 0.4 µM, and 0.4 µM, respectively, and subjected to real-time PCR.

Custom synthesized 18s MGB Probe (Applied Biosystems(R)): 5'-ATTGGAGGGCAAGTCTGGTGCCAGC-3' (SEQ ID NO: 5)
Custom synthesized 18s Primer 1 (Thermo Fisher Scientific, Inc.): 5'-CGGCTACCACATCCAAGGAAG-3' (SEQ ID NO: 6)
Custom synthesized 18s Primer 2 (Thermo Fisher Scientific, Inc.): 5'-GCTGGAATTACCGCGGCT-3' (SEQ ID NO: 7)

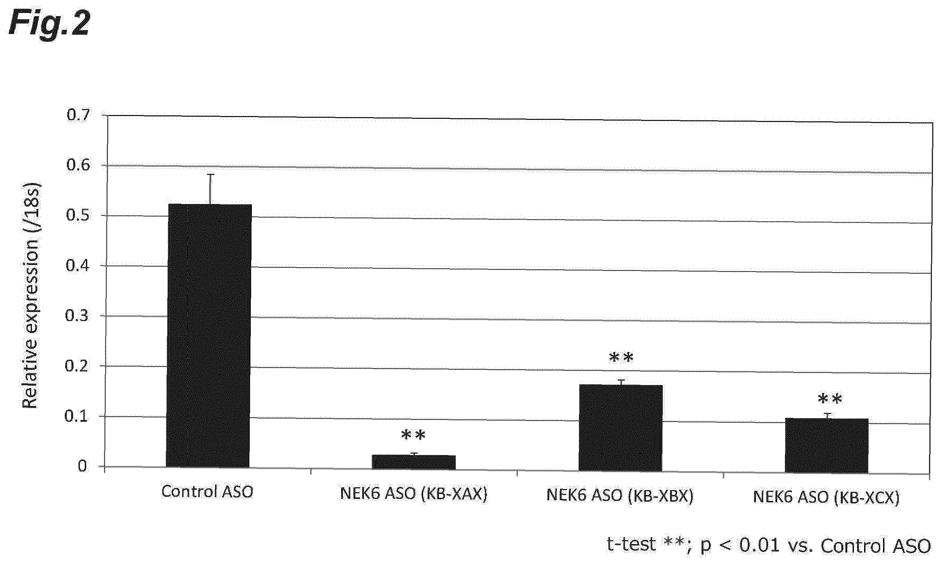

Figure 2 shows results of real-time PCR of NEK6 when NEK6 antisense oligonucleotide was introduced. Introduction of NEK6 antisense oligonucleotides suppressed the transcript amount of NEK6 gene. Consequently, it was shown that the NEK6 antisense oligonucleotides used suppressed efficiently the expression of a target gene.

### [Example 2: Influence of NEK6 knockdown on phosphorylation of SMAD3]

In order to investigate possibility that NEK6 antisense oligonucleotide may control TGF-β signal, the amount of phosphorylated SMAD3 was analyzed in cells transfected with each NEK6 antisense oligonucleotide.

Human NEK6 antisense oligonucleotides (KB-XAX, XBX, XCX) were transfected into human hepatic stellate cell line LI-90 cells using Lipofectamine RNAi MAX (Invitrogen(TM)) at a final concentration of 30 nM. 24 hours after transfection, the medium was changed from DMEM Low Glucose medium containing 10% FCS (Thermo Fisher Scientific, Inc.) to DMEM Low Glucose medium containing 0.2% FCS (Thermo Fisher Scientific, Inc.). 48 hours after transfection, human TGF-β protein (PeproTech, Inc.) was added so as to provide a final concentration of 5 ng/mL. 30 minutes after TGF-β addition, the cells were lysed with 2 × SDS sample buffer (100 mM Tris-HCl, pH 6.8, 4% SDS, 6 M Urea, 12% Glycerol, 2% protease inhibitor cocktail [Nacalai Tesque Inc.], 1% phosphatase inhibitor cocktail [Nacalai Tesque Inc.]) to give a cell extaction liquid.

To the cell extraction liquid thus obtained, β-Mercaptoethanol and Bromophenol blue were added so as to provide final concentrations of 5% and 0.025%, respectively, and then heated at 95°C for 4 minutes to give a sample. Using the sample thus obtained, SDS-PAGE was performed to separate proteins contained in the sample in accordance with their sizes. Then, the separated proteins were transferred onto a PVDF membrane, and subjected to Western blotting with an anti-phosphorylated SMAD3 antibody (Cell Signaling Technology, Inc.), and an anti-SMAD3 antibody (Cell Signaling Technology, Inc.), an anti-NEK6 antibody (Santa Cruz Biotechnology Inc.), and an anti-β-Actin antibody (Sigma-Aldrich Co. LLC.). Phosphorylated SMAD3 was calibrated by the total SMAD3 amount.

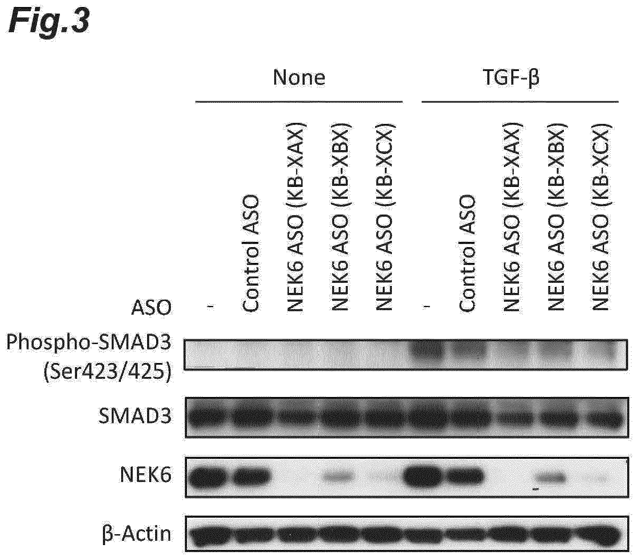

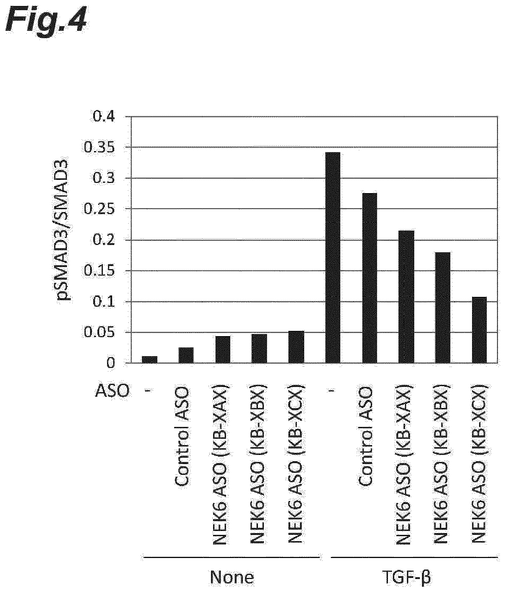

Figure 3 shows results of Western blot of phosphorylated SMAD3 protein when NEK6 was knocked down. Figure 4 shows the results of calibrating the phosphorylated SMAD3 amount by the total SMAD3 amount. By using NEK6 antisense oligonucleotides, the amount of NEK6 protein was decreased, and the amount of phosphorylated SMAD3 that is elevated by TGF-β was decreased. Consequently, it was shown that phosphorylation of SMAD3 protein is suppressed by NEK6 knockdown.

### [Example 3: Interaction between NEK6 protein and SMAD3 protein within a cell]

In order to investigate whether NEK6 protein would interact with SMAD3 and phosphorylates SMAD3 within a cell, co-immunoprecipitation was performed.

To human pulmonary fibroblast line LL29 cells established from the lung of an IPF patient, an expression vector in which human NEK6 was cloned (pEZ-M02 Nek6, GeneCopoeia, Inc.) and an expression vector in which FLAG-tag-labeled human SMAD3 was cloned (pEZ-M11 Flag-hSmad3, GeneCopoeia, Inc.) were transfected using X-treme GENE HP (Roche Diagnostics K. K). 24 hours after transfection, the medium was changed. 48 hours after transfection, the cells were recovered with lysis buffer (175 mM NaCl, 50 mM HEPES, pH 7.6, 0.1% NP40, 0.2 mM EDTA, pH 8.0, 1.4 mM β-Mcrcaptoethanol, 1% protease inhibitor cocktail, 1% phosphatase inhibitor cocktail), and supernatant was obtained by centrifugation. To the supernatant, TrueBlot Anti-Goat IgIP Beads (Rockland Immunochemicals Inc.) was added and subjected to centrifugation to eliminate non-specific binding. To the supernatant thus obtained, a normal goat IgG (Santa Cruz Biotechnology Inc.) as a control or an anti-NEK6 antibody was added and incubated at 4°C overnight, and then TrueBlot Anti-Goat IgIP Beads was added and incubated at 4°C for 4 hours. After centrifugation and removal of supernatant, the TrueBlot Anti-Goat IgIP Beads was washed with lysis buffer to eliminate non-specific binding. To the TrueBlot Anti-Goat IgIP Beads, 2 × SDS PAGE loading buffer (100 mM Tris-HCl, pH 6.8, 4% SDS, 20% Glycerol, 0.2% Bromophenol blue, 50 mM DTT) was added and heated at 95°C for 5 minutes, and coimmunoprecipitate contained in the supernatant was recovered. For the coimmunoprecipitate with the anti-NEK6 antibody, Western blotting was performed with an anti-SMAD3 antibody (Cell Signaling Technology, Inc.) or an anti-NEK6 antibody, and subjected to detection whether NEK6 protein and SMAD3 protein would be co-immunopresipitated.

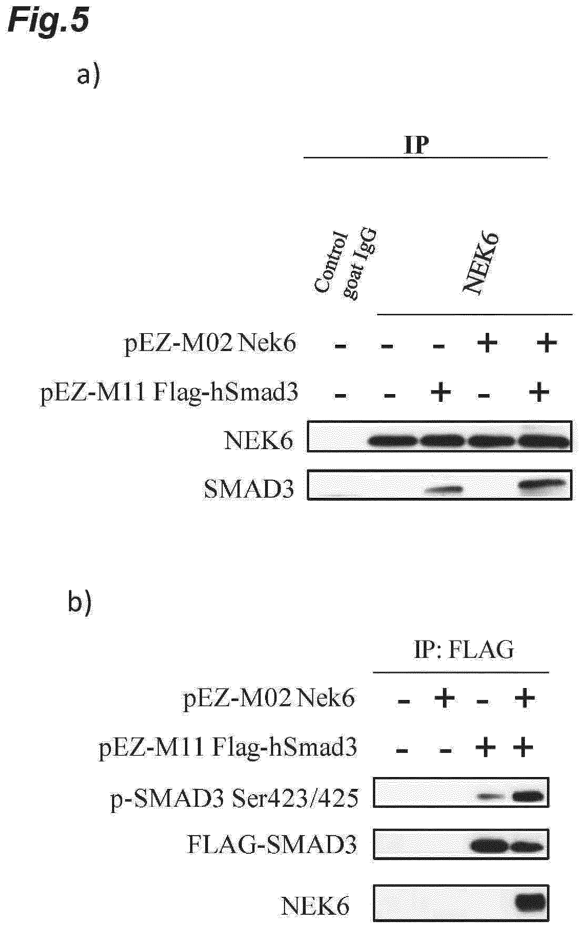

Figure 5a shows results of co-immunoprecipitation with the anti-NEK6 antibody. NEK6 protein and SMAD3 protein were detected from the coimmunoprecipitate.

In order to analyze whether SMAD3 protein interacts with NEK6 and is phosphorylated within a cell, an expression vector for human NEK6 and an expression vector for FLAG-tag-labeled human SMAD3 were transfected into LL29 cells. 24 hours after transfection, the medium was changed. 48 hours after transfection, the cells were recovered with lysis buffer (250 mM NaCl, 50 mM HEPES, pH 7.6, 0.1% NP40, 0.2 mM EDTA, pH8.0, 1.4 mM β-Mercaptoethanol, 1% protease inhibitor cocktail, 1% phosphatase inhibitor cocktail), and supernatant was obtained by centrifugation. To the supernatant thus obtained, Anti-FLAG M2 Affinity Gel (Sigma-Aldrich Co. LLC.) was added and incubated at 4°C overnight. Then centrifugation was performed to remove supernatant. The Anti-FLAG M2 Affinity Gel was washed with lysis buffer to eliminate non-specific binding. To the Anti-FLAG M2 Affinity Gel, 2 × SDS PAGE loading buffer (100 mM Tris-HCl, pH 6.8, 4% SDS, 20% Glycerol, 0.2% Bromophenol blue, 50 mM DTT) was added and heated at 95°C for 4 minutes, and coimmunoprecipitate contained in the supernatant was recovered. The coimmunoprecipitate thus obtained was separated with SDS-PAGE on the basis of the sizes, followed by transfer to a PVDF membrane, and subjected to Western blotting for the coimmunoprecipitate by Anti-FLAG M2 Affinity Gel using an anti-phosphorylated SMAD3 antibody, an anti-FLAG antibody (Sigma-Aldrich Co. LLC.), and an anti-NEK6 antibody, to detect whether NEK6 protein and phosphorylated SMAD3 protein would be co-immunoprecipitated.

Figure 5b shows results of co-immunoprecipitation by the anti-FLAG antibody. NEK6 protein and FLAG-SMAD3 protein were detected from coimmunoprecipitate. Moreover, transfection of a human NEK6 expression vector caused the amount of phosphorylated SMAD3 protein to be elevated.

Since SMAD3 protein was detected in coimmunoprecipitate by an anti-NEK6 antibody, and conversely, NEK6 was detected in coimmunoprecipitate by an anti-FLAG antibody, it was shown that NEK6 protein and SMAD3 protein interact within a cell and form a complex. Furthermore, since the amount of phosphorylated SMAD3 protein was elevated by transfection of a human NEK6 expression vector, it was shown that NEK6 protein phosphorylates SMAD3 protein within a cell.

### [Example 4: SMAD3 protein phosphorylation by NEK6 protein]

Possibility that NEK6 protein may directly phosphorylate SMAD3 protein as a substrate was investigated using purified proteins of NEK6 and SMAD3.

His-fusion NEK6 protein (Eurofins Scientific SE) and GST-fusion SMAD3 protein (Sigma-Aldrich Co. LLC.) were mixed with reaction solvent (150 µM ATP, 50 mM HEPES, 150 mM NaCl, 0.1% Triton X-100, 10 mM MgCl₂, 1 mM DTT, 1% phosphatase inhibitor cocktail), and incubated at 30°C for 45 minutes. To the reaction solution, 2 × SDS sample buffer containing 5% β-Mercaptoethanol and 0.025% Bromophenol blue were added in equal amounts to terminate the reaction, and then heated at 95°C for 5 minutes to give a sample. Using the sample thus obtained, SDS-PAGE was performed to separate proteins contained in the reaction solution in accordance with their sizes. Then, the separated proteins were transferred onto a PVDF membrane, and subjected to Western blotting with an anti-phosphorylated SMAD3 antibody and an anti-GST antibody (Santa Cruz Biotechnology Inc.), an anti-NEK6 antibody.

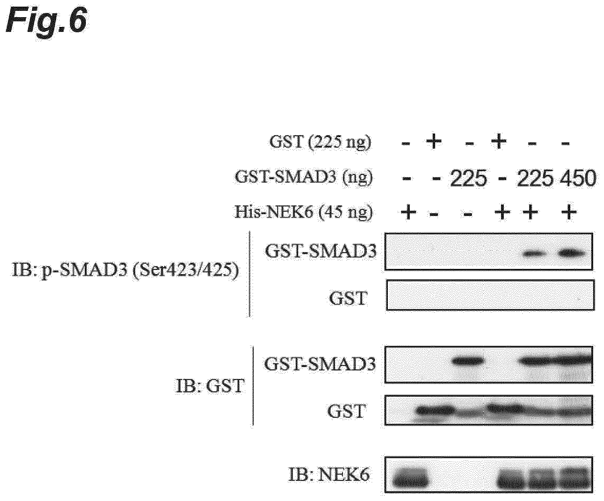

Figure 6 shows results of Western blot of phosphorylated SMAD3 protein, when His-fusion NEK6 protein and GST-fusion SMAD3 protein were reacted. By using NEK6 protein, the amount of phosphorylated SMAD3 was elevated. Consequently, it was shown that NEK6 protein directly phosphorylates SMAD3 protein as a substrate.

### [Example 5: Influence on transcriptional activity of SMAD protein complex by NEK6 knockdown]

In order to investigate whether NEK6 protein would also control transcriptional activity that generates after nuclear translocation of SMAD protein complex, a luciferase reporter assay with a DNA binding sequence of SMAD protein complex and luciferase gene was performed. Furthermore, Western blotting was performed using LL29 cells prepared simultaneously, to check for NEK6 knockdown.

To human pulmonary fibroblast line LL29 cells established from the lung of an IPF patient, ON-TARGET plus SMART pool siRNA (Dharmacon Inc.), which is an siRNA for human NEK6, was transfected using Lipofectamine RNAi MAX. 24 hours after transfection of the siRNA, the medium was changed from F-12K medium containing 10% FCS to F-12K medium containing 0.4% FCS. 48 hours after transfection of the siRNA, an expression vector in which a DNA binding sequence of SMAD protein complex (SMAD biding element [SBE]) and firefly luciferase gene (pTL-SBE-luc: 5'-AGTATGTCTAGACTGAAGTATGTCTAGACTGAAGTATGTCTA GACTGA-3' [SEQ ID NO: 8], Panomics Inc.) were cloned, and a vector for calibration for a reporter assay which contains wildtype Renilla luciferase (pRL-TK: Promega Corporation) were transfected using Lipofectamine LTX with PLUS reagent (Thermo Fisher Scientific Inc). Two hours after transfection of the expression vectors, human TGF-β protein was added so as to provide a final concentration of 10 ng/mL. 24 hours after addition of TGF-β, the cells were lysed with 2 × SDS sample buffer (100 mM Tris-HCl, pH 6.8, 4% SDS, 6 M Urea, 12% Glycerol, 2% protease inhibitor cocktail, 1% phosphatase inhibitor cocktail). Proteins contained in the cell extraction liquid thus obtained were separated by SDS-PAGE, followed by transfer of the proteins onto a PVDF membrane, and subjected to Western blotting with an anti-SMAD3 antibody, an anti-NEK6 antibody, and an anti-Vinculin antibody. Meanwhile, LL29 cells prepared in a similar manner as described above were recovered in accordance with Dual-Luci ferase Reporter Assay System (Promega Corporation), and luminescence by firefly luciferase and Renilla luciferase was measured. The luminescence quantity of firefly luciferase was calibrated by the luminescence quantity of Renilla luciferase.

### <ON-TARGET plus SMART pool siRNA>

siNEK6: 5'-CUGUCCUCGGCCUAUCUUC-3' (SEQ ID NO: 9)
siNEK6: 5'-UAUUUGGGUGGUUCAGUUG-3' (SEQ ID NO: 10)
siNEK6: 5'-CAACUCCAGCACAAUGUUC-3' (SEQ ID NO: 11)
siNEK6: 5'-UACUUGAUCAUCUGCGAGA-3' (SEQ ID NO: 12)

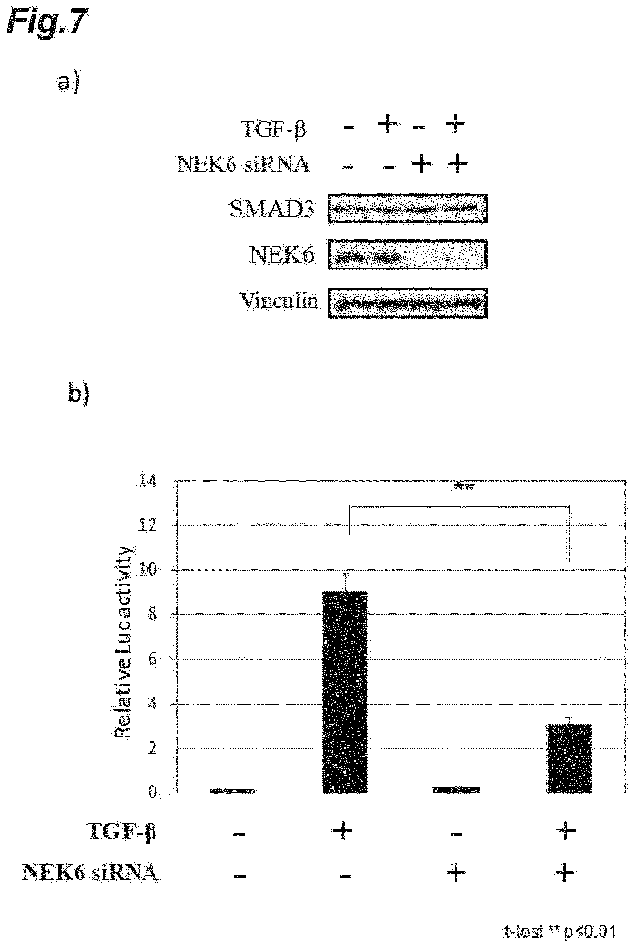

Figure 7a shows results of Western blot when NEK6 was knockdown. It was shown that by using NEK6 siRNAs, the amount of NEK6 protein decreased, but the amount of SMAD3 protein did not change. Figure 7b shows the luminescence quantity of firefly luciferase calibrated by that of Renilla luciferase when NEK6 was knockdown. By using NEK6 siRNAs, the luminescence quantity of firefly luciferase that is elevated by TGF-β was decreased. Consequently, it was shown that transcriptional activity of SMAD protein complex is suppressed by NEK6 knockdown.

### [Example 6: Influence on transcript amounts of fibrosis-related genes by NEK6 knockdown in hepatic stellate cells]

In order to investigate that NEK6 knockdown exhibits an efficacy against fibrosis, the transcript amounts of fibrosis-related genes were analyzed in cells transfected with NEK6 antisense oligonucleotides.

Human NEK6 antisense oligonucleotides (KB-XAX, XBX, and XCX) were transfected into human hepatic stellate cell line LI-90 cells at a final concentration of 30 nM using Lipofectamine RNAi MAX (Invitrogen(TM)). 48 hours after transfection, the medium was changed from DMEM Low Glucose medium containing 10% FCS (Thermo Fisher Scientific, Inc.) to DMEM Low Glucose medium containing 0.2% FCS (Thermo Fisher Scientific, Inc.). 72 hours after transfection, human TGF-β protein (PeproTech, Inc.) was added so as to provide a final concentration of 2 ng/ml. 24 hours after addition of TGF-β, RNAs were extracted from the cells transfected with NEK6 antisense oligonucleotides, using RNeasy Mini Kit (Qiagen).

The RNAs thus obtained were subjected to reverse transcription using High Capacity cDNA Reverse Transcription Kit (Applied Biosystems(R)) to obtain cDNAs. The cDNAs thus obtained were subjected to real-time PCR using TaqMan Gene Expression Assays (Applied Biosystems) to examine influence on the transcript amounts of genes by NEK6 knockdown. The transcript amounts of Col1a1 gene and Fibronectin gene were calculated by dividing a measurement value in Collal Taqman Probe (HS00164004_m1. Applied Biosystems(R)) or Fibronectin Taqman Probe (HS01549976_m1, Applid Biosystems) by a measurement value in 18s Probe. As the 18s Probe, the following custom synthesized 18s MGB Probe, custom synthesized 18s Primer 1, and custom synthesized 18s Primer 2 were mixed so as to be 0.2 µM, 0.4 µM, and 0.4 µM, respectively, and subjected to real-time PCR.

Custom synthesized 18s MGB Probe (Applied Biosystems(R)): 5'-ATTGGAGGGCAAGTCTGGTGCCAGC-3' (SEQ ID NO: 5)
Custom synthesized 18s Primer 1 (Thermo Fisher Scientific, Inc.): 5'-CGGCTACCACATCCAAGGAAG-3' (SEQ ID NO: 6)
Custom synthesized 18s Primer 2 (Thermo Fisher Scientific, Inc.): 5'-GCTGGAATTACCGCGGCT-3' (SEQ ID NO: 7)

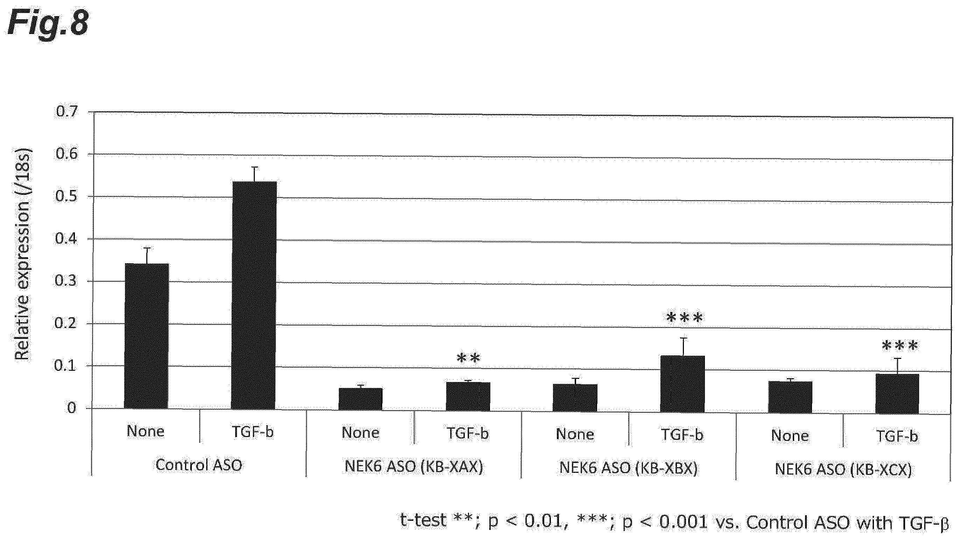

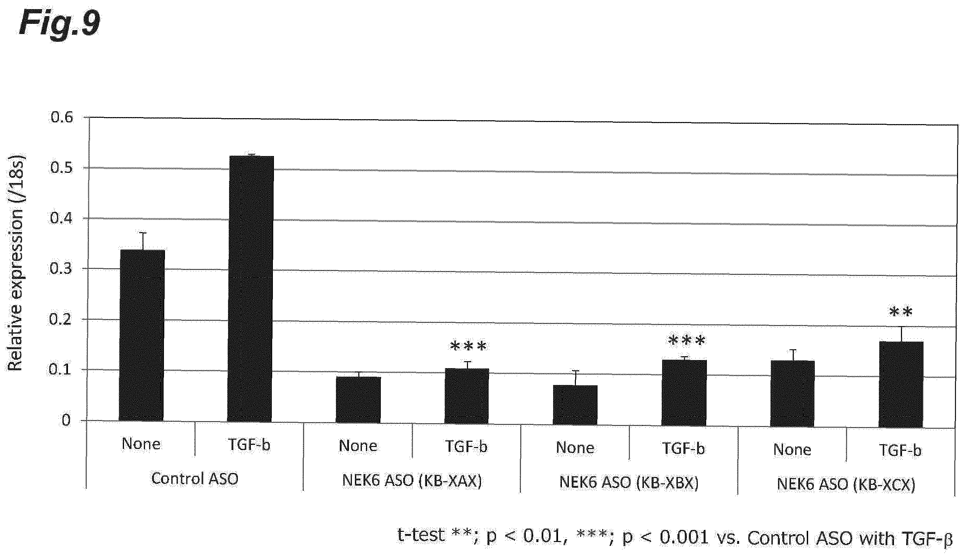

Figure 8 shows the transcript amount of Collal gene when NEK6 was knocked down, and Figure 9 shows the transcript amount of Fibronectin gene when NEK6 was knocked down. By NEK6 antisense oligonucleotides, the transcript amounts of Collal and Fibronectin genes that are elevated by TGF-β were decreased. Consequently, it was shown that NEK6 knockdown suppresses fibrogenesis.

### [Example 7: Influence on transcript amounts of fibrosis-related genes by NEK6 knockdown in pulmonary fibroblasts]

In order to investigate that NEK6 knockdown exhibits an efficacy against fibrosis, the transcript amounts of fibrosis-related genes were analyzed in cells transfected with NEK6 siRNAs.

To human pulmonary fibroblast line LL29 cells established from the lung of an IPF patient, an siRNA for human NEK6 (ON-TARGET plus SMART pool siRNA, Dharmacon Inc.) was transfected using Lipofectamine RNAi MAX. 24 hours after transfection, the medium was changes from F-12K medium containing 10% FCS to F-12K medium containing 0.1% BSA. 48 hours after transfection, human TGF-β protein was added so as to provide a final concentration of 1 ng/mL. 72 hours after transfection, RNAs were extracted from the cells transfected with the siRNA, using RNeasy Mini Kit. The RNAs thus obtained were subjected to reverse transcription using High Capacity cDNA Reverse Transcription Kit to give cDNAs. The cDNAs thus obtained were subjected to real-time PCR using TaqMan Gene Expression Assays to detect influence on the transcript amounts of genes by NEK6 knockdown. The transcript amounts of Collal gene and αSMA gene were calculated by dividing a measurement value in Collal Taqman Probe (HS00164004_ml, Applied Biosystems(R)) or aSMA Taqman Probe (HS00426835_gl, Applied Biosystems(R)) by a measurement value in 18s Probe.

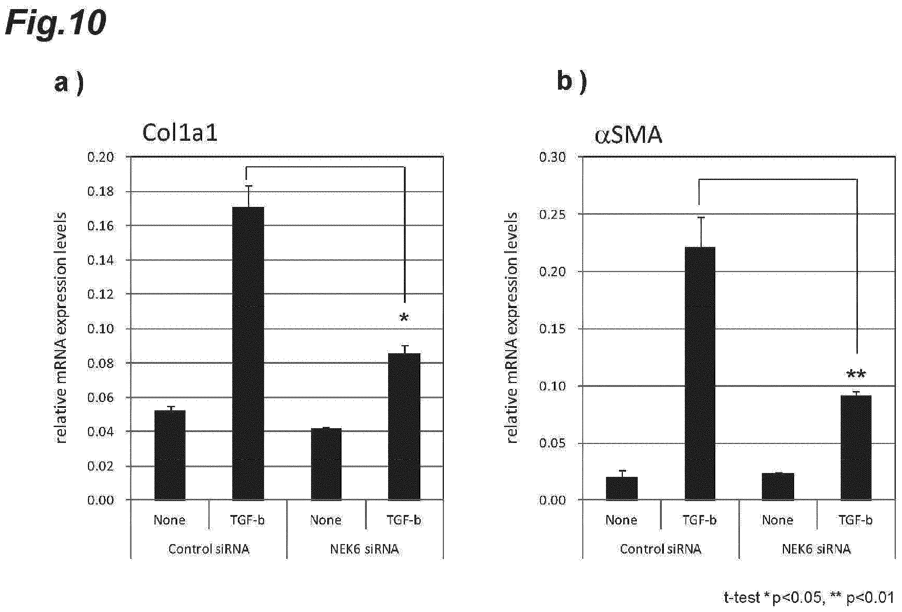

Figure 10a shows the transcript amount of Collal when NEK6 was knockdown, and Figure 10b shows the transcript amount of aSMA gene when NEK6 was knockdown. By using NEK6 siRNAs, the transcript amounts of Collal and aSMA genes that are elevated by TGF-β were decreased. Consequently, it was shown that NEK6 knockdown suppresses fibrosis.

### [Example 8: Influence of NEK6 knockdown on phosphorylation of SMAD3 protein in kidney fibroblasts]

In order to investigate possibility that NEK6 protein may control TGF-β signal, the amount of phosphorylated SMAD3 was analyzed in cells transfected with NEK6 siRNAs.

To rat kidney fibroblast line NRK-49F cells, ssPN molecule for human NEK6 (KB-004: 5'-GAUAAGAUGAAUCUCUUCUCCCC-Lx-GGGGAGAAGAGAU UCAUCUUAUCUC-3' (SEQ ID NO: 16), the underlines represent sequences suppressing expression) were transfected using Lipofectamine RNAi MAX. 24 hours after transfection, the medium was changed from DMEM medium containing 10% FCS to DMEM medium containing 0.1% FCS. 48 hours after transfection, human TGF-β protein was added so as to provide a final concentration of 5 ng/ml. Thirty minutes or an hour after addition, the cell were lysed with 2 × SDS sample buffer (100 mM Tris-HCl, pH 6.8, 4% SDS, 6 M Urea, 12% Glycerol, 2% protease inhibitor cocktail, 1% phosphatase inhibitor cocktail) to give a cell extraction liquid. To the cell etraction liquid thus obtained, β-Mcrcaptoethanol and Bromophenol blue were added so as to provide final concentrations of 5% and 0.025%, respectively, and then heated at 95°C for 4 minutes to give a sample. Using the sample thus obtained, SDS-PAGE was performed to separate proteins contained in the sample in accordance with their sizes. Then, the separated proteins were transferred onto a PVDF membrane, and subjected to Western blotting with an anti-phosphorylated SMAD3 antibody and an anti-SMAD3 antibody, an anti-NEK6 antibody (Abcam plc.), and an anti-Vinculin antibody.

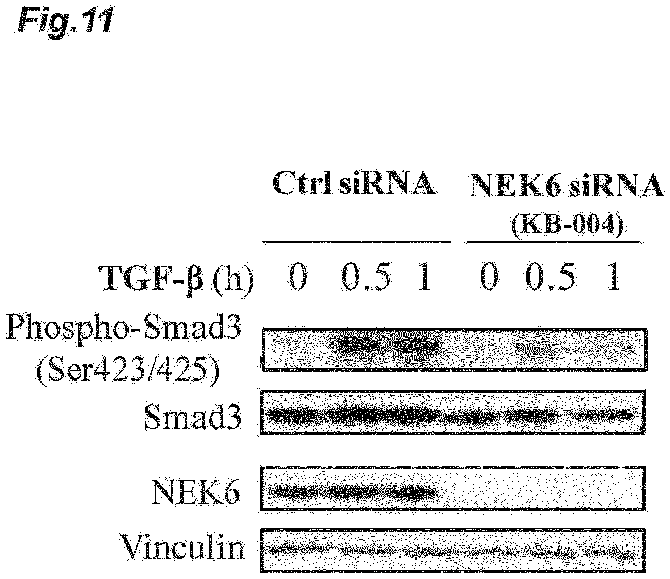

Figure 11 shows results of Western blotting of phosphorylated SMAD3 protein when NEK6 was knockdown. By using NEK6 siRNAs, the amount of NEK6 protein was decreased, and the amount of phosphorylated SMAD3 that is elevated by TGF-β was decreased. Consequently, it was shown that SMAD3 protein phosphorylation is suppressed by NEK6 knockdown.

### [Example 9: Evaluation of efficacy of NEK6 knockdown in carbon tetrachloride (CCl₄)-induced hepatic fibrogenesis models]

In order to check that NEK6 knockdown exhibits efficacy against fibrosis, NEK6 siRNAs were intravenously administered into CCl₄ model mice and subjected to analysis of anti-fibrogenesis action.

To 7-weeks-old male C57BL/6J mice (Charles River Laboratories Japan, Inc.), olive oil solution containing 10 v/v% CCl₄ (FUJIFILM Wako Pure Chemical Corporation) was intraperitoneally administered at 10 mL/kg by body weight on day 0, 4, 7, and 11 to create hepatic fibrogenesis model mice. Grouping was performed with body weight at the day before the initial administration of CCl₄, and the design of the groups was as a saline administration group not receiving CCl₄ (n = 5), a solvent-administration group receiving CCl₄ (n = 10), and nucleic acid administration group receiving CCl₄ (n = 10). Using KB-004 as NEK6 ssPN molecules and Invivofectamine 3.0 Reagent (Thermo Fisher Scientific Inc.) as an administration solvent, 0.3 mg/mL of nucleic acid administration solution was made according to the product protocol of Invivofectamine 3.0. For the nucleic acid administration group, the nucleic acid administration solution containing KB-004 was administered via tail vein so as to provide 3 mg/kg by body weight; and for the solvent administration group, the administration solvent in an equal amount to that of the nucleic acid administration group was administered via tail vein on the day before the initial administration of CCl₄ and day 10 after induction of pathology. Evaluation of hepatic disorder and fibrogenesis was performed on day 13 after induction of pathology (Examples 10, 11, 12, and 14).

### [Example 10: Analysis of hepatic disorder markers in CCl₄ models]

Fibrogenesis has been understood as an excessive wound healing process against disorder of a cell or tissue. Thus, suppression of disorder of a cell or tissue along with fibrogenesis has been considered to be effective for treatment of various fibrosis. Then, in order to investigate whether NEK6 knockdown would exhibit effect on hepatic disorder, measurement of hepatic disorder markers in CCl₄ models was performed.

On day 13 after induction of pathology, blood draw was performed from tail vein using a plane capillary blood-sampling tube, and subjected to standing for 30 minutes or more. The post-standing blood was centrifuged to obtain serum. Serum glutamic pyruvic transaminase (GPT) and glutamic oxaloacetic transaminase (GOT) were measured using Transaminase CII-test Wako (Wako Pure Chemical Industries, Ltd.). Measurement method followed the instruction of the reagent.

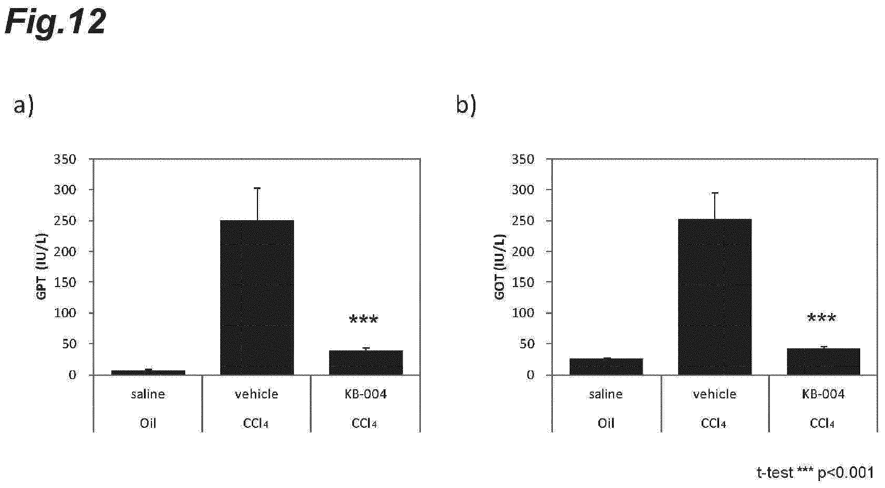

Figure 12a has shown measurement results of serum GPT, and Figure 12b has shown measurement results of serum GOT. Elevation of serum GPT and GOT found in CCl₄ models was suppressed by administering NEK6 siRNAs. Consequently, it was shown that NEK6 knockdown suppresses hepatic disorder.

### [Example 11: Analysis of SMAD3 protein phosphorylation in CCl₄ models]

In order to investigate whether NEK6 knockdown would exhibit effect on SMAD3 protein phosphorylation, the amount of phosphorylated SMAD3 in CCl₄ models was analyzed.

A liver collected on day 13 after induction of pathology was frozen and grinded to be powdery. To the powdery liver, lysis buffer (150 mM NaCl, 1% NP40, 0.1% SDS, 50 mM Tris-HCl, pH7.5, 1 mM EDTA, 1 mM Benzylsulfonyl fluoride, 2% protease inhibitor cocktail, 1% phosphatase inhibitor cocktail) was added, and an organ extract was prepared using a handy ultrasonic generator. To the supernatant obtained by centrifuging the organ extract, β-Mercaptoethanol and Bromophenol blue were added so as to provide final concentrations of 5% and 0.025%, respectively. Then, heating was made at 95°C for 4 minutes to give a sample. Using the sample thus obtained, SDS-PAGE was performed to separate proteins contained in the sample in accordance with their sizes. Then, the separated proteins were transferred onto a PVDF membrane, and subjected to Western blotting with an anti-phosphorylated SMAD3 antibody (Abcam plc.) and an anti-SMAD3 antibody, an anti-NEK6 antibody (Abcam plc.), and an anti-Vinculin antibody. Phosphorylated SMAD3 was calibrated by the total SMAD3 amount.

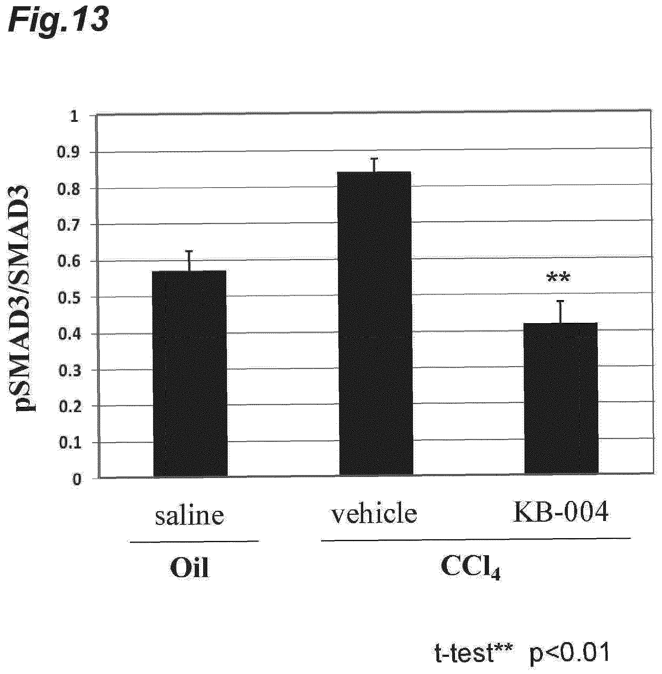

Figure 13 shows results of Western blotting of phosphorylated SMAD3 protein when NEK6 was knockdown. By using NEK6 siRNAs, the amount of NEK6 protein was decreased, and the amount of phosphorylated SMAD3 that is elevated by TGF-β was decreased. Consequently, it was shown that NEK6 knockdown suppresses SMAD3 protein phosphorylation in the liver of a CCl₄ model.

### [Example 12: Analysis of fibrosis-related genes in CCl₄ models]

In order to investigate whether NEK6 knockdown would exhibit efficacy against fibrogenesis, the transcript amounts of fibrosis-related genes in CCl₄ models were analyzed.

RNAs were extracted from a liver collected on day 13 after induction of pathology, using QIAzol Lysis reagent (QIAGEN N.V). Subsequently, RNAs were purified using RNeasy mini kit, and subjected to reverse transcription reaction using High-Capacity cDNA Reverse Transcription Kit. The transcript amounts of NEK6 Taqman Probe (Mm00480730_ml, Applied Biosystems(R)); and Collal Taqman Probe (Mm00801666_gl, Applied Biosystems(R)), Col3a1 Taqman Probe (Mm01254476_ml, Applied Biosystems(R)), and Timp1 Taqman Probe (Mm01341361_m1, Applied Biosystems(R)) as fibrosis-related genes were measured using TaqMan Gene Expression Assay, and relative ratios to the transcript amount of 18s rRNA Taqman Probe (Hs99999901_s1, Applied Biosystems(R)), which is an inner control, is defined as the transcript amount of each gene.

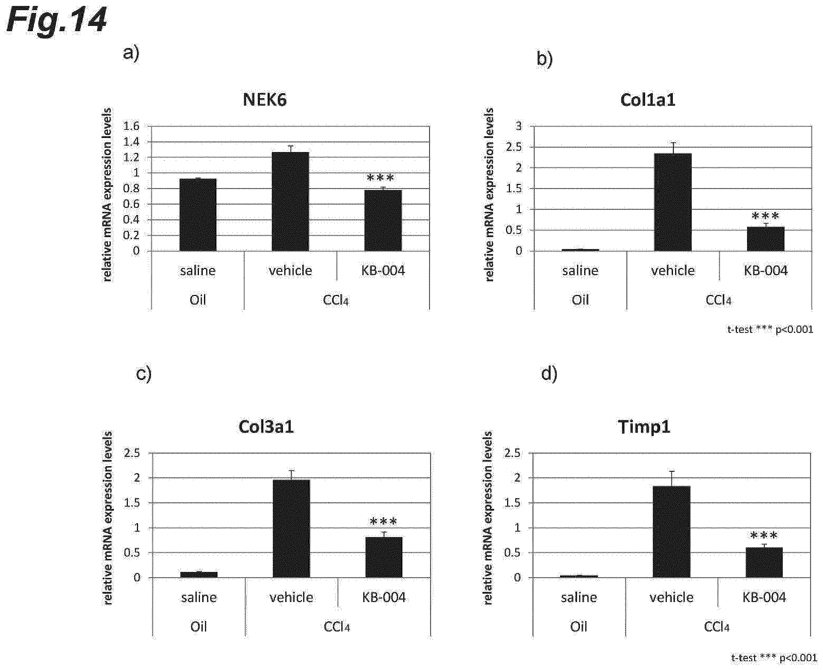

The transcript amounts of each gene in CCl₄ models are shown in Figures 14a-d. The transcript amount of NEK6 gene decreased by administration of NEK6 ssPN molecules. From this, it was shown that KB-004 used suppressed efficiently target gene transcription. At this time, the transcript amounts of fibrosis-related genes (Col1a1, Col3a1, Timp1) that are derived by induction of pathology significantly decreased. Consequently, it was shown that NEK6 knockdown suppresses fibrogenesis.

### [Example 13: Analysis of fibrosis-related genes in bile duct ligation-induced hepatic fibrogenesis (BDL) models]

In order to investigate that NEK6 knockdown exhibits efficacy against fibrosis, NEK6 siRNAs were intravenously administered into bile duct ligation-induced hepatic fibrogenesis model mice (BDL models) to analyze the transcript amounts of fibrosis-related genes.

Nine-weeks-old C57BL/6J mice (Charles River Laboratories Japan, Inc.) were grouped in accordance with body weight, and KB-004 solution (0.3 mg/mL) prepared according to the package insert of the gene transfer reagent Invivofectamine 3.0 (Thermo Fisher Scientific Inc.) was administered via tail vein at a dose of 3 mg/kg by body weight (n = 12). For a control group, only solvent was administered (n = 15). The next day of administration, the common bile duct was ligated at two points to create a hepatic fibrogenesis model mouse. For a sham-operated group, saline (Otsuka Normal Saline) was administered via tail vein, and only detachment of the common bile duct was performed (n = 7). A liver was collected on day 14 after bile duct ligation; and the transcript amounts of NEK6 Taqman Probe (Mm00480730_m1. Applied Biosystems(R)), and Collal Taqman Probe (Mm00801666_g1, Applied Biosystems(R)), Col3a1 Taqman Probe (Mm01254476_m1. Applied Biosystems(R)), and Timp1 Taqman Probe (Mm01341361_m1, Applied Biosystems(R)) as fibrosis-related genes were measured in a similar manner as described above, and relative ratios to the transcript amount of GAPDH Taqman Probe (Mm9999995_g1, Applied Biosystems(R)), which is an inner control, is defined as the transcript amount of each gene.

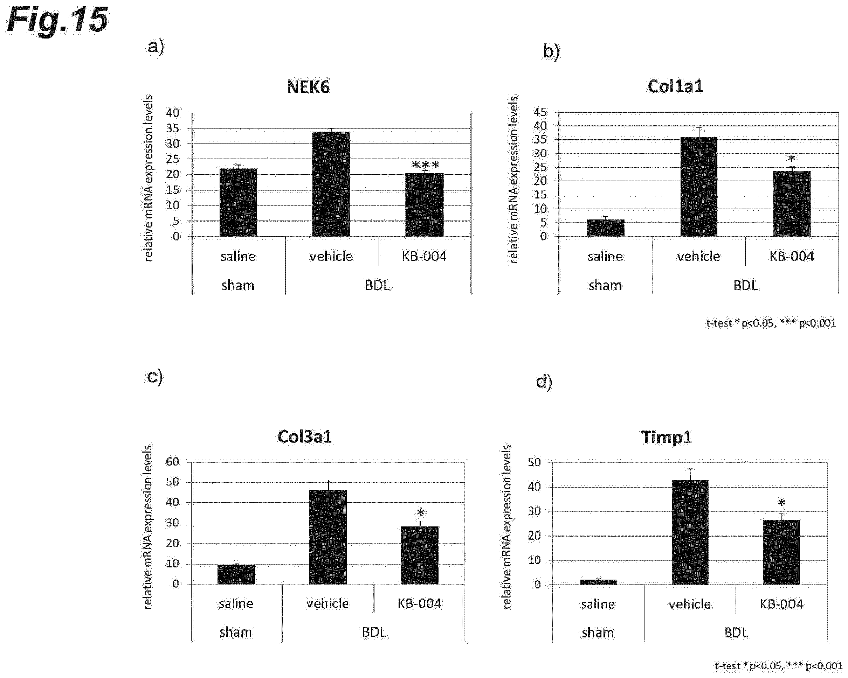

Each gene transcript amount in BDL models are shown in Figures 15a-d. The transcript amount of NEK6 gene decreased by administration of NEK6 siRNAs. From this, it was shown that KB-004 used suppressed efficiently target gene transcription. At this time, the transcript amounts of fibrosis-related genes (Collal, Col3a1, Timp1) that are derived by induction of pathology significantly decreased. Consequently, it was shown that NEK6 knockdown suppresses fibrogenesis.

### [Example 14: Pathological analysis in CCl₄ models]

In order to investigate whether NEK6 knockdown would exhibit effect against a CCl₄-induced hepatic fibrogenesis model, observation of histopathology was performed.

The inner right lobe of a liver was collected on day 13 after induction of pathology, and fixed with 10% neutral buffered formalin solution. After embedding with paraffin, tissue sections were made and subjected to hematoxylin-eosin staining.

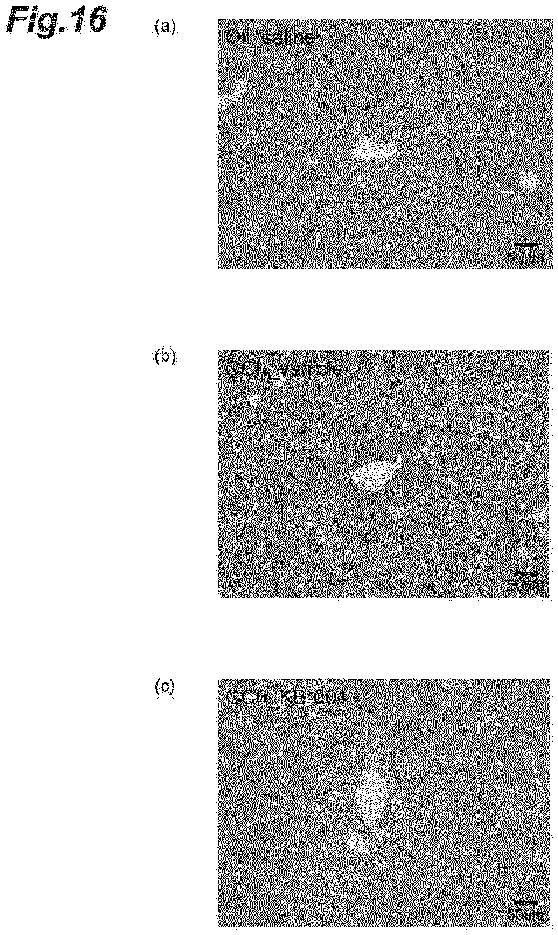

Figure 16 shows representative examples of histopathology. Figure 16a represents results of the saline administration group not receiving CCl₄, Figure 16b represents results of the solvent administration group receiving CCl₄, and Figure 16c represents results of the nucleic acid administration group receiving CCl₄. Vacuolar degeneration numerously found in Figure 16b indicates cell disorder, and an area that is abundantly present around the central part and in which the nucleuses are not stained indicates cell necrosis. Disorder, necrosis, and the like of cells found in a liver tissue of a CCl₄ model were decreased by administering NEK6 siRNAs. Consequently, it was shown that NEK6 knockdown suppresses change of histopathology in a CCl₄-induced hepatic fibrogenesis model.

### Reference example 1: Synthesis of single-strand nucleic acid molecules

The nucleic acid molecules shown below were synthesized on the basis of a phosphoroamidite method with a nucleic acid synthesizer (trade name: ABI3900 DNA Synthesizer, Applied Biosystems). Solid-phase synthesis was performed using controlled pore glass (CPG) as a solid-phase carrier, and EMM amidite (WO2013/027843) as RNA amidite. Excision from the solid-phase carrier and deprotection of a phosphate group protecting group, deprotection of a nucleotide protecting group, and deprotection of a 2'-hydroxyl group protecting group followed conventional methods. The synthesized single-strand nucleic acid molecules were purified by HPLC.

In the following single-strand nucleic acid molecules of the present invention, Lx is a linker region Lx, and represents L-proline diamide amidite of the following structural formula.

### [Chemical Formula 1]

Additionally, the underlines in the following single-strand nucleic acid molecules represent sequences that suppress NEK6 gene expression.
KB-001 
5'-GAGGGAGUUCCAACAACCUCUCC-Lx-GGAGAGGUUGUUG
GAACUCCCUCCA-3' (SEQ ID NO: 13)
KB-002 
5'-CGAGGCAGGACUGUGUCAAGGCC-Lx-GGCCUUGACACAG
UCCUGCCUCGCC-3' (SEQ ID NO: 14)
KB-003 
5'-CGUGGAGCACAUGCAUUCACGCC-Lx-GGCGUGAAUGCAU
GUGCUCCACGGC-3' (SEQ ID NO: 15)
KB-004 
5'-GAUAAGAUGAAUCUCUUCUCCCC-Lx-GGGGAGAAGAGAU
UCAUCUUAUCUC-3' (SEQ ID NO: 16)
KB-005

5'-CAGAGACCUGACAUCGGAUACCC-Lx-GGGUAUCCGAUGUC
AGGUCUCUGGU-3' (SEQ ID NO: 17)

## Claims

1. A phosphorylation inhibitor of SMAD2/3 protein, comprising an antisense nucleic acid that suppresses NEK6 gene expression as an active ingredient.

2. A therapeutic agent for fibrosis, comprising an antisense nucleic acid that suppresses NEK6 gene expression as an active ingredient.

3. The therapeutic agent according to claim 2, wherein the fibrosis is pulmonary fibrosis, hepatic fibrosis, or kidney fibrosis.

4. The inhibitor according to claim 1, wherein the antisense nucleic acid that suppresses NEK6 gene expression comprises a sequence selected from the group consisting of a sequence having at least 90% sequence identity to a sequence of SEQ ID NO: 1, a sequence having at least 90% sequence identity to a sequence of SEQ ID NO: 2, and a sequence having at least 90% sequence identity to a sequence of SEQ ID NO: 3.

5. The inhibitor according to claim 1, wherein the antisense nucleic acid that suppresses NEK6 gene expression comprises a sequence selected from the group consisting of a sequence of SEQ ID NO: 1, a sequence of SEQ ID NO: 2, and a sequence of SEQ ID NO: 3.
